# EUROPEAN PATENT SPECIFICATION

(11) **EP 1 141 324 B1**
(45) Date of publication and mention of the grant of the patent: **27.09.2006**
(21) Application number: 99960735.1
(22) Date of filing: 16.12.1999
(51) Int. Cl.: C12N 15/54, C12N 15/82, A01H 5/00, A01H 5/10, A01H 3/00

(54) **DIACYLGLYCEROL ACYLTRANSFERASE GENE FROM PLANTS**
DIACYLGLYZERIN-ACYLTRANSFERASE GEN AUS PFLANZEN
GENE DE LA DIACYLGLYCEROL ACYLTRANSFERASE D'ORIGINE VEGETALE

(30) Priority: 17.12.1998 US 112812 P
(43) Date of publication of application: 10.10.2001
(73) Proprietor: NATIONAL RESEARCH COUNCIL OF CANADA, Ottawa, Ontario K1A OR6 (CA)
(72) Inventor: ZOU, Jitao, Saskatoon, Saskatchewan S7H 4T8 (CA); TAYLOR, David, C., Saskatoon, Saskatchewan S7J 4C3 (CA); WEI, Yangdou, Saskatoon, Saskatchewan S7H 3A7 (CA); JAKO, Colette, C., Saskatoon, Saskatchewan S7K 2W3 (CA)
(74) Representative: Hallybone, Huw George
(86) International application number: PCT/CA1999/001202
(87) International publication number: WO 2000/036114

(56) References cited:
- WO-A-00/01713
- WO-A-99/63096
- WO-A-99/67403
- R61u012 Database Entry Ac005917 Accession number AC005917; 4 November 1998 LIN X. ET AL.:"Arabidopsis thaliana chromosome II section 113 of 255 of the complete sequence" XP002133608 -& LIN X. ET AL. : "Sequence and analysis of chromosome II of Arabidopsis thaliana" NATURE, vol. 402, 1999, pages 761-768, XP000877287 LONDON GB
- CASES S ET AL: "Identification of a gene encoding an acyl CoA: diacylglycerol acyltransferase, a key enzyme in triacylglycerol synthesis" PROCEEDINGS OF THE NATIONAL ACADEMY OF SCIENCES OF USA,US,NATIONAL ACADEMY OF SCIENCE. WASHINGTON, vol. 95, no. 22, 27 October 1998 (1998-10-27), pages 13018-13023, XP002122745 ISSN: 0027-8424 cited in the application
- VESNA KATAVIC ET AL.: "Alteration of seed fatty acid composition by an ethyl methanesulfonate-induced mutation in Arabidopsis thaliana affecting diacylglycerol acyltransferase activity" PLANT PHYSIOLOGY, vol. 108, 1995, pages 399-409, XP002915657 cited in the application
- Empln Database Entry Ath238008 Accession number AJ238008; 18 June 1999 ZOU J. ET AL.:"The Arabidopsis thaliana TAG1 gene encodes for a diacylglycerol acyltransferase" XP002133609 cited in the application
- ZOU, JITAO ET AL: "The Arabidopsis thaliana TAG1 mutant has a mutation in a diacylglycerol acyltransferase gene" PLANT J. (1999), 19(6), 645-653 , XP002133607
- EMBL Database Entry AF155224 Accession number AF155224; 30 June 1999 NYKIFORUK C.L. ET AL.:"Brassica napus putative diacylglycerol cyltransferase (DGAT2) mRNA" XP002133639 cited in the application & NYKIFORUK C.L. ET AL.: "Isolation and sequence analysis of a novel cDNA encoding a putative diacylglycerol acyltransferase from a microspore-derived cell suspension culture of Brassica napus L. cv Jet Neuf (Accession No. AF155224) (PGR99-123)." PLANT PHYSIOLOGY, vol. 120, no. 4, 1999, pages 1207-1207, LONDON GB
- EMBL Database Entry AF164434 Accession number AF164434; 26 July 1999 NYKIFORUK C.L. ET AL.:"Brassica napus putative diacylglycerol acyltransferase (DGAT1) mRNA" XP002133640 cited in the application & NYKIFORUK C.L. ET AL.: "Isolation and characterization of a cDNA encoding a second putative diacylglycerol acyltransferase from a microspore-derived cell suspension culture of Brassica napus L. cv Jet Neuf (Accession No. AF164434) (PGR99-158)" PLANT PHYSIOLOGY, vol. 121, no. 3, 1999, pages 1053-1053,
- HOBBS D H ET AL: "Cloning of a cDNA encoding diacylglycerol acyltransferase from Arabidopsis thaliana and its functional expression" FEBS LETTERS,NL,ELSEVIER SCIENCE PUBLISHERS, AMSTERDAM, vol. 452, no. 3, 11 June 1999 (1999-06-11), pages 145-149, XP002122747 ISSN: 0014-5793
- Database Entry Ac005917; Accession number AC005917; 4 November 1998; LIN X. ET AL: 'Arabidopsis thaliana IGF BAC F3P11 genomic sequence near marker CIC06E08 (issued 16.09.1998)'
- Database Entry Ac005917; Accession number AC005917; SV AC005917.1 November 1998; LIN X. ET AL: 'Arabidopsis thaliana chromosome II BAC F3P11 genomic sequence (issued 15.02.1999)'

### SEQUENCE LISTING

<110> National Research Council of Canada
   Zou, Jitao
   Taylor, David C
   Wei, Yangdou
   Jako, Colette C
<120> Diacylglycerol Acyl Transferase Gene from Plants
<130> 43922pt
<140> PCT/CA
   <141> 1999-12-16
<150> 60/112,812
   <151> 1998-12-17
<160> 25
<170> PatentIn Ver. 2.1
<210> 1
   <211> 1904
   <212> DNA
   <213> Arabidopsis thaliana
<400> 1
   atttcttagc ttcttccttc aatccgctct ttccctctcc attagattct gtttcctctt 60
   tcaatttctt ctgcatgctt ctcgattctc tctgacgcct cttttctccc gacgctgttt 120
   cgtcaaacgc ttttcgaaat ggcgattttg gattctgctg gcgttactac ggtgacggag 180
   aacggtggcg gagagttcgt cgatcttgat aggcttcgtc gacggaaatc gagatcggat 240
   tcttctaacg gacttcttct ctctggttcc gataataatt ctccttcgga tgatgttgga 300
   gctcccgccg acgttaggga tcggattgat tccgttgtta acgatgacgc tcagggaaca 360
   gccaatttgg ccggagataa taacggtggt ggcgataata acggtggtgg aagaggcggc 420
   ggagaaggaa gaggaaacgc cgatgctacg tttacgtatc gaccgtcggt tccagctcat 480
   cggagggcga gagagagtcc acttagctcc gacgcaatct tcaaacagag ccatgccgga 540
   ttattcaacc tctgtgtagt agttcttatt gctgtaaaca gtagactcat catcgaaaat 600
   cttatgaagt atggttggtt gatcagaacg gatttctggt ttagttcaag atcgctgcga 660
   gattggccgc ttttcatgtg ttgtatatcc ctttcgatct ttcctttggc tgcctttacg 720
   gttgagaaat tggtacttca gaaatacata tcagaacctg ttgtcatctt tcttcatatt 780
   attatcacca tgacagaggt tttgtatcca gtttacgtca ccctaaggtg tgattctgct 840
   tttttatcag gtgtcacttt gatgctcctc acttgcattg tgtggctaaa gttggtttct 900
   tatgctcata ctagctatga cataagatcc ctagccaatg cagctgataa ggccaatcct 960
   gaagtctcct actacgttag cttgaagagc ttggcatatt tcatggtcgc tcccacattg 1020
   tgttatcagc caagttatcc acgttctgca tgtatacgga agggttgggt ggctcgtcaa 1080
   tttgcaaaac tggtcatatt caccggattc atgggattta taatagaaca atatataaat 1140
   cctattgtca ggaactcaaa gcatcctttg aaaggcgatc ttctatatgc tattgaaaga 1200
   gtgttgaagc tttcagttcc aaatttatat gtgtggctct gcatgttcta ctgcttcttc 1260
   cacctttggt taaacatatt ggcagagctt ctctgcttcg ggatcgtgaa attctacaaa 1320
   gattggtgga atgcaaaaag tgtgggagat tactggagaa tgtggaatat gcctgttcat 1380
   aaatggatgg ttcgacatat atacttcccg tgcttgcgca gcaagatacc aaagacactc 1440
   gccattatca ttgctttcct agtctctgca gtctttcatg agctatgcat cgcagttcct 1500
   tgtcgtctct tcaagctatg ggcttttctt gggattatgt ttcaggtgcc tttggtcttc 1560
   atcacaaact atctacagga aaggtttggc tcaacggtgg ggaacatgat cttctggttc 1620
   atcttctgca ttttcggaca accgatgtgt gtgcttcttt attaccacga cctgatgaac 1680
   cgaaaaggat cgatgtcatg aaacaactgt tcaaaaaatg actttcttca aacatctatg 1740
   gcctcgttgg atctccgttg atgttgtggt ggttctgatg ctaaaacgac aaatagtgtt 1800
   ataaccattg aagaagaaaa gaaaattaga gttgttgtat ctgcaaaaat tttggtagag 1860
   acacgcaaac ccgtttggat tttgttatgg tgtaaagcgg ccgc                 1904
<210> 2
   <211> 520
   <212> PRT
   <213> Arabidopsis thaliana
<400> 2
   Met Ala Ile Leu Asp Ser Ala Gly Val Thr Thr Val Thr Glu Asn Gly
    1               5                  10                  15
   Gly Gly Glu Phe Val Asp Leu Asp Arg Leu Arg Arg Arg Lys Ser Arg
               20                  25                  30
   Ser Asp Ser Ser Asn Gly Leu Leu Leu Ser Gly Ser Asp Asn Asn Ser
           35                  40                  45
   Pro Ser Asp Asp Val Gly Ala Pro Ala Asp Val Arg Asp Arg Ile Asp
       50                  55                  60
   Ser Val Val Asn Asp Asp Ala Gln Gly Thr Ala Asn Leu Ala Gly Asp
   65                  70                  75                  80
   Asn Asn Gly Gly Gly Asp Asn Asn Gly Gly Gly Arg Gly Gly Gly Glu
                   85                  90                  95
   Gly Arg Gly Asn Ala Asp Ala Thr Phe Thr Tyr Arg Pro Ser Val Pro
               100                 105                 110
   Ala His Arg Arg Ala Arg Glu Ser Pro Leu Ser Ser Asp Ala Ile Phe
           115                 120                 125
   Lys Gln Ser His Ala Gly Leu Phe Asn Leu Cys Val Val Val Leu Ile
       130                 135                 140
   Ala Val Asn Ser Arg Leu Ile Ile Glu Asn Leu Met Lys Tyr Gly Trp
   145                 150                 155                 160
   Leu Ile Arg Thr Asp Phe Trp Phe Ser Ser Arg Ser Leu Arg Asp Trp
                   165                 170                 175
   Pro Leu Phe Met Cys Cys Ile Ser Leu Ser Ile Phe Pro Leu Ala Ala
               180                 185                 190
   Phe Thr Val Glu Lys Leu Val Leu Gln Lys Tyr Ile Ser Glu Pro Val
           195                 200                 205
   Val Ile Phe Leu His Ile Ile Ile Thr Met Thr Glu Val Leu Tyr Pro
       210                 215                 220
   Val Tyr Val Thr Leu Arg Cys Asp Ser Ala Phe Leu Ser Gly Val Thr
   225                 230                 235                 240
   Leu Met Leu Leu Thr Cys Ile Val Trp Leu Lys Leu Val Ser Tyr Ala
                   245                 250                 255
   His Thr Ser Tyr Asp Ile Arg Ser Leu Ala Asn Ala Ala Asp Lys Ala
               260                 265                 270
   Asn Pro Glu Val Ser Tyr Tyr Val Ser Leu Lys Ser Leu Ala Tyr Phe
           275                 280                 285
   Met Val Ala Pro Thr Leu Cys Tyr Gln Pro Ser Tyr Pro Arg Ser Ala
       290                 295                 300
   Cys Ile Arg Lys Gly Trp Val Ala Arg Gln Phe Ala Lys Leu Val Ile
   305                 310                 315                 320
   Phe Thr Gly Phe Met Gly Phe Ile Ile Glu Gln Tyr Ile Asn Pro Ile
                   325                 330                 335
   Val Arg Asn Ser Lys His Pro Leu Lys Gly Asp Leu Leu Tyr Ala Ile
               340                 345                 350
   Glu Arg Val Leu Lys Leu Ser Val Pro Asn Leu Tyr Val Trp Leu Cys
           355                 360                 365
   Met Phe Tyr Cys Phe Phe His Leu Trp Leu Asn Ile Leu Ala Glu Leu
       370                 375                 380
   Leu Cys Phe Gly Asp Arg Glu Phe Tyr Lys Asp Trp Trp Asn Ala Lys
   385                 390                 395                 400
   Ser Val Gly Asp Tyr Trp Arg Met Trp Asn Met Pro Val His Lys Trp
                   405                 410                 415
   Met Val Arg His Ile Tyr Phe Pro Cys Leu Arg Ser Lys Ile Pro Lys
               420                 425                 430
   Thr Leu Ala Ile Ile Ile Ala Phe Leu Val Ser Ala Val Phe His Glu
           435                 440                 445
   Leu Cys Ile Ala Val Pro Cys Arg Leu Phe Lys Leu Trp Ala Phe Leu
       450                 455                 460
   Gly Ile Met Phe Gln Val Pro Leu Val Phe Ile Thr Asn Tyr Leu Gln
   465                 470                 475                 480
   Glu Arg Phe Gly Ser Thr Val Gly Asn Met Ile Phe Trp Phe Ile Phe
                   485                 490                 495
   Cys Ile Phe Gly Gln Pro Met Cys Val Leu Leu Tyr Tyr His Asp Leu
               500                 505                 510
   Met Asn Arg Lys Gly Ser Met Ser
           515                 520
<210> 3
   <211> 5193
   <212> DNA
   <213> Arabidopsis thaliana
<400> 3
   gctcacgacc cattcttccc gttccatttg gttttattta tttcaaagtt taatattcct 60
   tttgtataac attcaaatct tcacatgatt gattgtgtga aaaccccaca gattttacta 120
   caataggggg agttgactta aaatagctat tgatgtcgaa aaaatgtatt ttagttataa 180
   attatactaa agaaaatttt tgatttgtct gttgtttaag catatgtatt gttaaactta 240
   aaaaaatatg tattgttaat cttaaaaatg taggagtaca catcaaatac tcgagcataa 300
   tcaaaaccgt attcatagac cgatgtgaga atcaaataga agataatgtg attttttaaa 360
   atatcgtatc tccaaatcaa tcacttagaa gataatgtaa ttctttatgt gctacataaa 420
   taaatatata tatatatata tatatatatc ttgtatatat gtcttgacaa aaaattgcca 480
   gtcaaaaacc atgactgaat caaactataa gtcggattga atcaaactat aagtcggatg 540
   agtattaatt tccattatgt ttctatactt tacaaaccgg aaaatagata ttatagatac 600
   caaaaaagta gatttgtgta tattattaga agatttggaa tttcatcatt atcaggatct 660
   aaagtacttc cctaattaaa tcatgtcggt tgaaaaagct caatgaatgt ttgaaatttg 720
   gaaagtttat taaattcgga tctttttttt ttgtttgtcg tcccaaacat ttttatttta 780
   ttacaaataa tcaacttatc cttactacta aatcatttca tatctttgat accaacaaat 840
   catttcatat tctattttga tgtttaagaa aacactattt accagttaca aaatattata 900
   aggattgttg tttagaaaaa aaagtacaag ttgaattctt tttgtcaaat ataaaattga 960
   ctttttaata tataattgac ttattgaaca tgattacaga attaatcatc tacaaaactt 1020
   tccaagttta taataaatac atttcaaaga ctattagttc ttcttaaaat atttctaaaa 1080
   gtgatcaaag actaccacat ataattcaga aaaagtagaa gttgatttct ttttgtcaaa 1140
   taaataattg acttaaaata gtttggaaag ccattgaact tgattataga attgataatg 1200
   tacataaaaa aattccaagt ttataataaa tacatttttc aaatgctata tcagttcttc 1260
   ttaaaatatt tcactaaaaa aacactcaaa tatagaataa atttattgaa taacatacca 1320
   actgtaaaac agaatttgac aaaaaaaaaa aaaaaatgaa atgaagatga agacaaaaat 1380
   aaatcaccag aggatcttat gcaaaaaaat atatgaatac acaataaacc atattgatat 1440
   ttttaaaata aaataaaaac agaaaaatat cccaacaccg cttttcaatt aaaaatcttc 1500
   cgtcaccatt gttgtcatct tcctctctcg tgaatccttt ttcctttctt cttcttcttc 1560
   tcttcagaga aaactttgct tctctttcta taaggaacca gacacgaatc ccattcccac 1620
   cgatttctta gcttcttcct tcaatccgct ctttccctct ccattagatt ctgtttcctc 1680
   tttcaatttc ttctgcatgc ttctcgattc tctctgacgc ctcttttctc ccgacgctgt 1740
   ttcgtcaaac gcttttcgaa atggcgattt tggattctgc tggcgttact acggtgacgg 1800
   agaacggtgg cggagagttc gtcgatcttg ataggcttcg tcgacggaaa tcgagatcgg 1860
   attcttctaa cggacttctt ctctctggtt ccgataataa ttctccttcg gatgatgttg 1920
   gagctcccgc cgacgttagg gatcggattg attccgttgt taacgatgac gctcagggaa 1980
   cagccaattt ggccggagat aataacggtg gtggcgataa taacggtggt ggaagaggcg 2040
   gcggagaagg aagaggaaac gccgatgcta cgtttacgta tcgaccgtcg gttccagctc 2100
   atcggagggc gagagagagt ccacttagct ccgacgcaat cttcaaacag gtttaaaatc 2160
   tcagaaatct tcgaatttgg tgtttgcttg ttgttttata tggaattgag tttggtgatt 2220
   gttttgcatt gcagagccat gccggattat tcaacctctg tgtagtagtt cttattgctg 2280
   taaacagtag actcatcatc gaaaatctta tgaaggtttg ctgttacttg tttctccttt 2340
   taggaattga attgcttgaa aatttatcag agacgaataa ctttgttgtt gctatcattc 2400
   atgtagtatg gttggttgat cagaacggat ttctggttta gttcaagatc gctgcgagat 2460
   tggccgcttt tcatgtgttg gtaaaagaag atgtttttta tttccagcaa tgttacattg 2520
   ttatacgtat aatgatgagt ttagtgatca agttcctctt tgattcttct ttcttgttgc 2580
   agtatatccc tttcgatctt tcctttggct gcctttacgg ttgagaaatt ggtacttcag 2640
   aaatacatat cagaacctgt gagtaattac tattctccag ccattactgt aatttttatt 2700
   gaagacaagt ttgtatcatg aagaacttac aagttctgtt ttgaaaatgc tcaaggttgt 2760
   catctttctt catattatta tcaccatgac agaggttttg tatccagttt acgtcaccct 2820
   aaggtgatac tgtttttctg gtctcagttt gtgatactgt ttttaagttt agttgtctga 2880
   cccggtgatc ttgaaaatgg acaggtgtga ttctgctttt ttatcaggtg tcactttgat 2940
   gctcctcact tgcattgtgt ggctaaagtt ggtttcttat gctcatacta gctatgacat 3000
   aagatcccta gccaatgcag ctgataaggt aaaatacgaa aaagaagcgt atgtattagt 3060
   cacttgcact gtgttactgt tttaaccaaa cactgttatg aactttaggc caatcctgaa 3120
   gtctcctact acgttagctt gaagagcttg gcatatttca tggtcgctcc cacattgtgt 3180
   tatcaggtaa ctgcaaagtg catcaaccat tcttatactt gcaagagttt cttgtctaaa 3240
   cctcggatct ttgcttttcc ccagccaagt tatccacgtt ctgcatgtat acggaagggt 3300
   tgggtggctc gtcaatttgc aaaactggtc atattcaccg gattcatggg atttataata 3360
   gaacaagtac gttttcacat cttgctttat tagttttcct tggtgaaaat catcatccct 3420
   gcgttgtcac cacttgactt catgttcttt tgttacattt tggcagtata taaatcctat 3480
   tgtcaggaac tcaaagcatc ctttgaaagg cgatcttcta tatgctattg aaagagtgtt 3540
   gaagctttca gttccaaatt tatatgtgtg gctctgcatg ttctactgct tcttccacct 3600
   ttggtatgct gtgatcccat ctctttcaaa ataatttgca aattcgaaaa accgaaaaag 3660
   gctaaatctc atacgaattt gatattttta gtttcttaga gtcggtgatg taatttcagt 3720
   tactgaacgc aaatctcttg tccaaaggtt aaacatattg gcagagcttc tctgcttcgg 3780
   ggatcgtgaa ttctacaaag attggtggaa tgcaaaaagt gtgggagatg tgagctattt 3840
   tactcaaaag aaaacttatg atttttaatg ttgtcgttgt ttttgggtca tctaactaac 3900
   caaattcatg tattcactgt cttcctttat cagtactgga gaatgtggaa tatggtatgg 3960
   ttctcttcct aaacatcacc ttcttttgta cacaaaatag aagaagagag ctaattaaga 4020
   tcttgttttc cttgacagcc tgttcataaa tggatggttc gacatatata cttcccgtgc 4080
   ttgcgcagca agataccaaa ggtgagtgag atatataccg atatgcaatt gtcgagattt 4140
   gtttctgtga tataaattta accctccaca cacttgtttt tcagacactc gccattatca 4200
   ttgctttcct agtctctgca gtctttcatg aggtatacat actttctaca ttgccctgtc 4260
   tctagacgca tgaacacacg ctagtgaaag aaatgctaat attcaaagca ttgtttttac 4320
   ttaacgatct tgtgttacaa atttcctttt gacagctatg catcgcagtt ccttgtcgtc 4380
   tcttcaagct atgggctttt cttgggatta tgtttcaggt taaaaaatta ctaaactgct 4440
   gcagtcgatt tttactaaac tctaatctca tattctgacc aaccaatttg tttgagtagg 4500
   tgcctttggt cttcatcaca aactatctac aggaaaggtt tggctcaacg gtatgctctc 4560
   aaaacccgag aaaatagaac gaataactct ttctttcata gcctagccat ttaaatcgca 4620
   atgctgaaac ttaataataa aggtgatctg ttttggaatg ggatcatatt attaggtggg 4680
   gaacatgatc ttctggttca tcttctgcat tttcggacaa ccgatgtgtg tgcttcttta 4740
   ttaccacgac ctgatgaacc gaaaaggatc gatgtcatga aacaactgtt caaaaaatga 4800
   ctttcttcaa acatctatgg cctcgttgga tctccgttga tgttgtggtg gttctgatgc 4860
   taaaacgaca aatagtgtta taaccattga agaagaaaag aaaattagag ttgttgtatc 4920
   tgcaaaaatt ttggtagaga cacgcgaacc cgtttggatt ttgttatggt gtaaagaaat 4980
   ttcaatcaaa aaactgttgt aataattgtt accaaaaaga aatgcttttc tggaaacgag 5040
   gggaaaaata gtagttttgt taggttttac tgtttggacc aaatctagta aaaaactttt 5100
   tgtaataagg aaaaaaaaag aacaaatgtg ataaatgcat ggggattgta tgaaaccttc 5160
   caataaagtt gattggtggt cccgttttgg gga                              5193
<210> 4
   <211> 498
   <212> PRT
   <213> mouse
<400> 4
   Met Gly Asp Arg Gly Gly Ala Gly Ser Ser Arg Arg Arg Arg Thr Gly
   1               5                   10                  15
   Ser Arg Val Ser Val Gln Gly Gly Ser Gly Pro Lys Val Glu Glu Asp
               20                  25                  30
   Glu Val Arg Asp Ala Ala Val Ser Pro Asp Leu Gly Ala Gly Gly Asp
           35                  40                  45
   Ala Pro Ala Pro Ala Pro Ala Pro Ala His Thr Arg Asp Lys Asp Gly
       50                  55                  60
   Arg Thr Ser Val Gly Asp Gly Tyr Trp Asp Leu Arg Cys His Arg Leu
   65 70 75 80
   Gln Asp Ser Leu Phe Ser Ser Asp Ser Gly Phe Ser Asn Tyr Arg Gly
   85 90 95
   Ile Leu Asn Trp Cys Val Val Met Leu Ile Leu Ser Asn Ala Arg Leu
   100 105 110
   Phe Leu Glu Asn Leu Ile Lys Tyr Gly Ile Leu Val Asp Pro Ile Gln
   115 120 125
   Val Val Ser Leu Phe Leu Lys Asp Pro Tyr Ser Trp Pro Ala Pro Cys
   130 135 140
   Val Ile Ile Ala Ser Asn Ile Phe Val Val Ala Ala Phe Gln Ile Glu
   145 150 155 160
   Lys Arg Leu Ala Val Gly Ala Leu Thr Glu Gln Met Gly Leu Leu Leu
   165 170 175
   His Val Val Asn Leu Ala Thr Ile Ile Cys Phe Pro Ala Ala Val Ala
   180 185 190
   Leu Leu Val Glu Ser Ile Thr Pro Val Gly Ser Val Phe Ala Leu Ala
   195 200 205
   Ser Tyr Ser Ile Met Phe Leu Lys Leu Tyr Ser Tyr Arg Asp Val Asn
   210 215 220
   Leu Trp Cys Arg Gln Arg Arg Val Lys Ala Lys Ala Val Ser Thr Gly
   225 230 235 240
   Lys Lys Val Ser Gly Ala Ala Ala Gln Gln Ala Val Ser Tyr Pro Asp
   245 250 255
   Asn Leu Thr Tyr Arg Asp Leu Tyr Tyr Phe Ile Phe Ala Pro Thr Leu
   260 265 270
   Cys Tyr Glu Leu Asn Phe Pro Arg Ser Pro Arg Ile Arg Lys Arg Phe
   275 280 285
   Leu Leu Arg Arg Val Leu Glu Met Leu Phe Phe Thr Gln Leu Gln Val
   290 295 300
   Gly Leu Ile Gln Gln Trp Met Val Pro Thr Ile Gln Asn Ser Met Lys
   305 310 315 320
   Pro Phe Lys Asp Met Asp Tyr Ser Arg Ile Ile Glu Arg Leu Leu Lys
   325 330 335
   Leu Ala Val Pro Asn His Leu Ile Trp Leu Ile Phe Phe Tyr Trp Phe
   340 345 350
   Phe His Ser Cys Leu Asn Ala Val Ala Glu Leu Leu Gln Phe Gly Asp
   355 360 365
   Arg Glu Phe Tyr Arg Asp Trp Trp Asn Ala Glu Ser Val Thr Tyr Phe
       370                 375                 380
   Trp Gln Asn Trp Asn Ile Pro Val His Lys Trp Cys Ile Arg His Phe
   385                 390                 395                 400
   Tyr Lys Pro Met Leu Arg His Gly Ser Ser Lys Trp Val Ala Arg Thr
                   405                 410                 415
   Gly Val Phe Leu Thr Ser Ala Phe Phe His Glu Tyr Leu Val Ser Val
               420                 425                 430
   Pro Leu Arg Met Phe Arg Leu Trp Ala Phe Thr Ala Met Met Ala Gln
           435                 440                 445
   Val Pro Leu Ala Trp Ile Val Gly Arg Phe Phe Gln Gly Asn Tyr Gly
       450                 455                 460
   Asn Ala Ala Val Trp Val Thr Leu Ile Ile Gly Gln Pro Val Ala Val
   465                 470                 475                 480
   Leu Met Tyr Val His Asp Tyr Tyr Val Leu Asn Tyr Asp Ala Pro Val
                   485                 490                 495
   Gly Val
<210> 5
   <211> 488
   <212> PRT
   <213> human
<400> 5
   Met Gly Asp Arg Gly Ser Ser Arg Arg Arg Arg Thr Gly Ser Arg Pro
    1               5                  10                  15
   Ser Ser His Gly Gly Gly Gly Pro Ala Ala Ala Glu Glu Glu Val Arg
               20                  25                  30
   Asp Ala Ala Ala Gly Pro Asp Val Gly Ala Ala Gly Asp Ala Pro Ala
           35                  40                  45
   Pro Ala Pro Asn Lys Asp Gly Asp Ala Gly Val Gly Ser Gly His Trp
       50                  55                  60
   Glu Leu Arg Cys His Arg Leu Gln Asp Ser Leu Phe Ser Ser Asp Ser
   65                  70                  75                  80
   Gly Phe Ser Asn Tyr Arg Gly Ile Leu Asn Trp Cys Val Val Met Leu
                   85                  90                  95
   Ile Leu Ser Asn Ala Arg Leu Phe Leu Glu Asn Leu Ile Lys Tyr Gly
               100                 105                 110
   Ile Leu Val Asp Pro Ile Gln Val Val Ser Leu Phe Leu Lys Asp Pro
           115                 120                 125
   His Ser Trp Pro Ala Pro Cys Leu Val Ile Ala Ala Asn Val Phe Ala
       130                 135                 140
   Val Ala Ala Phe Gln Val Glu Lys Arg Leu Ala Val Gly Ala Leu Thr
   145                 150                 155                 160
   Glu Gln Ala Gly Leu Leu Leu His Val Ala Asn Leu Ala Thr Ile Leu
                   165                 170                 175
   Cys Phe Pro Ala Ala Val Val Leu Leu Val Glu Ser Ile Thr Pro Val
               180                 185                 190
   Gly Ser Leu Leu Ala Leu Met Ala His Thr Ile Leu Phe Leu Lys Leu
           195                 200                 205
   Phe Ser Tyr Arg Asp Val Asn Ser Trp Cys Arg Arg Ala Arg Ala Lys
       210                 215                 220
   Ala Ala Ser Ala Gly Lys Lys Ala Ser Ser Ala Ala Ala Pro His Thr
   225                 230                 235                 240
   Val Ser Tyr Pro Asp Asn Leu Thr Tyr Arg Asp Leu Tyr Tyr Phe Leu
                   245                 250                 255
   Phe Ala Pro Thr Leu Cys Tyr Glu Leu Asn Phe Pro Arg Ser Pro Arg
               260                 265                 270
   Ile Arg Lys Arg Phe Leu Leu Arg Arg Ile Leu Glu Met Leu Phe Phe
           275                 280                 285
   Thr Gln Leu Gln Val Gly Leu Ile Gln Gln Trp Met Val Pro Thr Ile
       290                 295                 300
   Gln Asn Ser Met Lys Pro Phe Lys Asp Met Asp Tyr Ser Arg Ile Ile
   305                 310                 315                 320
   Glu Arg Leu Leu Lys Leu Ala Val Pro Asn His Leu Ile Trp Leu Ile
                   325                 330                 335
   Phe Phe Tyr Trp Leu Phe His Ser Cys Leu Asn Ala Val Ala Glu Leu
               340                 345                 350
   Met Gln Phe Gly Asp Arg Glu Phe Tyr Arg Asp Trp Trp Asn Ser Glu
           355                 360                 365
   Ser Val Thr Tyr Phe Trp Gln Asn Trp Asn Ile Pro Val His Lys Trp
       370                 375                 380
   Cys Ile Arg His Phe Tyr Lys Pro Met Leu Arg Arg Gly Ser Ser Lys
   385                 390                 395                 400
   Trp Met Ala Arg Thr Gly Val Phe Leu Ala Ser Ala Phe Phe His Glu
                   405                 410                 415
   Tyr Leu Val Ser Val Pro Leu Arg Met Phe Arg Leu Trp Ala Phe Thr
               420                 425                 430
   Gly Met Met Ala Gln Ile Pro Leu Ala Trp Phe Val Gly Arg Phe Phe
           435                 440                 445
   Gln Gly Asn Tyr Gly Asn Ala Ala Val Trp Leu Ser Leu Ile Ile Gly
       450                 455                 460
   Gln Pro Ile Ala Val Leu Met Tyr Val His Asp Tyr Tyr Val Leu Asn
   465                 470                 475                 480
   Tyr Glu Ala Pro Ala Ala Glu Ala
                   485
<210> 6
   <211> 629
   <212> DNA
   <213> Arabidopsis thaliana
<400> 6
   tgcatgtata cggaagggtt gggtggctcg tcaatttgca aaactggtca tattcaccgg 60
   attcatggga tttataatag aacaatatat aaatcctatt gtcaggaact caaagcatcc 120
   tttgaaaggc gatcttctat atgctattga aagagtgttg aagctttcag ttccaaattt 180
   atatgtgtgg ctctgcatgt tctactgctt cttccacctt tggttaaaca tattggcaga 240
   gcttctctgc ttcggggatc gtgaattcta caaagattgg tggaatgcaa aaagtgtggg 300
   agattactgg gagaatgtgg aatatgcctg tccataaatg ggatgggtcc gacatatata 360
   ccttccccgt gcttgcgcac aaggattacc caaagacacc ccggccatta accattggct 420
   ttcccaagcc ccctggaggc ctttccatgg gccanggacc cggngtnccc tggcnggccc 480
   ttcaaagcaa agggggnttn cctggggnta aagntccang ggcccttggg gcccanccaa 540
   aannttcccc cgggaaaggg ttgcccaccg gggggngaaa aanncccggg ggcaccncgg 600
   aattttggga acccgggggg ggccttttt                                  629
<210> 7
   <211> 11
   <212> PRT
   <213> Arabidopsis thaliana
<400> 7
   His Lys Trp Met Val Arg His Ile Tyr Phe Pro
   1               5                   10
<210> 8
   <211> 341
   <212> PRT
   <213> Brassica napus
<400> 8
   Met Cys Cys Leu Ser Leu Ser Ile Phe Pro Leu Ala Ala Phe Thr Val
   1               5                   10                  15
   Glu Lys Leu Val Leu Gln Lys Cys Ile Ser Glu Pro Val Val Ile Phe
               20                  25                  30
   Leu His Val Ile Ile Thr Met Thr Glu Val Leu Tyr Pro Val Tyr Val
           35                  40                  45
   Thr Leu Arg Cys Asp Ser Ala Phe Leu Ser Gly Asp Thr Leu Met Leu
       50                  55                  60
   Leu Thr Cys Ile Val Trp Leu Lys Leu Val Ser Tyr Ala His Thr Asn
   65                  70                  75                  80
   Tyr Asp Ile Arg Thr Leu Ala Asn Ser Ser Asp Lys Ala Asn Pro Glu
                   85                  90                  95
   Val Ser Tyr Tyr Val Ser Leu Lys Ser Leu Ala Tyr Phe Met Leu Ala
               100                 105                 110
   Pro Thr Leu Cys Tyr Gln Pro Ser Tyr Pro Arg Ser Pro Cys Ile Arg
           115                 120                 125
   Lys Gly Trp Val Ala Arg Gln Phe Ala Lys Leu Val Ile Phe Thr Gly
       130                 135                 140
   Leu Met Gly Phe Ile Ile Glu Gln Tyr Ile Asn Pro Ile Val Arg Asn
   145                 150                 155                 160
   Ser Lys His Pro Leu Lys Gly Asp Leu Leu Tyr Ala Ile Glu Arg Val
                   165                 170                 175
   Leu Lys Leu Ser Val Pro Asn Leu Tyr Val Trp Leu Cys Met Phe Tyr
               180                 185                 190
   Cys Phe Phe His Leu Trp Leu Asn Ile Leu Ala Glu Leu Leu Cys Phe
           195                 200                 205
   Gly Asp Arg Glu Phe Tyr Lys Asp Trp Trp Asn Ala Lys Ser Val Gly
       210                 215                 220
   Asp Tyr Trp Arg Met Trp Asn Met Pro Val His Lys Trp Met Val Arg
   225                 230                 235                 240
   His Val Tyr Phe Pro Cys Leu Arg Ile Lys Ile Pro Lys Val Pro Ala
                   245                 250                 255
   Ile Ile Ile Ala Phe Leu Val Ser Ala Val Phe His Glu Leu Cys Ile
               260                 265                 270
   Ala Val Pro Cys Arg Leu Phe Asn Leu Trp Ala Phe Met Gly Ile Met
           275                 280                 285
   Phe Gln Val Pro Leu Val Phe Ile Thr Asn Phe Leu Gln Glu Arg Phe
       290                 295                 300
   Gly Ser Met Val Gly Asn Met Ile Phe Gly Ser Ala Ser Cys Ile Phe
   305                 310                 315                 320
   Gly Gln Pro Met Cys Gly Leu Leu Tyr Tyr His Asp Leu Met Asn Arg
   325 330 335
   Lys Gly Ser Met Ser
   340
<210> 9
   <211> 503
   <212> PRT
   <213> Brassica napus
<400> 9
   Met Ala Ile Leu Asp Ser Gly Gly Val Ala Val Pro Pro Thr Glu Asn
   1 5 10 15
   Gly Val Ala Asp Leu Asp Arg Leu His Arg Arg Lys Ser Ser Ser Asp
   20 25 30
   Ser Ser Asn Gly Leu Leu Ser Asp Thr Ser Pro Ser Asp Asp Val Gly
   35 40 45
   Ala Ala Ala Ala Glu Arg Asp Arg Val Asp Ser Ala Ala Glu Glu Glu
   50 55 60
   Ala Gln Gly Thr Ala Asn Leu Ala Gly Gly Asp Ala Glu Thr Arg Glu
   65 70 75 80
   Ser Ala Gly Gly Asp Val Arg Phe Thr Tyr Arg Pro Ser Val Pro Ala
   85 90 95
   His Arg Arg Thr Arg Glu Ser Pro Leu Ser Ser Asp Ala Ile Phe Lys
   100 105 110
   Gln Ser His Ala Gly Leu Phe Asn Leu Cys Val Val Val Leu Val Ala
   115 120 125
   Val Asn Ser Arg Leu Ile Ile Glu Asn Leu Met Lys Tyr Gly Trp Leu
   130 135 140
   Ile Arg Thr Asp Phe Trp Phe Ser Ser Thr Ser Leu Arg Asp Trp Pro
   145 150 155 160
   Leu Phe Met Cys Cys Leu Ser Leu Ser Val Phe Pro Leu Ala Ala Phe
   165 170 175
   Thr Val Glu Lys Met Val Leu Gln Lys Phe Ile Ser Glu Pro Val Ala
   180 185 190
   Ile Ile Leu His Val Ile Ile Thr Met Thr Glu Val Leu Tyr Pro Val
   195 200 205
   Tyr Val Thr Leu Arg Cys Asp Ser Ala Phe Leu Ser Gly Val Thr Leu
   210 215 220
   Met Leu Leu Thr Cys Ile Val Trp Leu Lys Leu Val Ser Tyr Ala His
   225                 230                 235                 240
   Thr Ser Tyr Asp Ile Arg Thr Leu Ala Asn Ser Ala Asp Lys Val Asp
                   245                 250                 255
   Pro Glu Ile Ser Tyr Tyr Val Ser Leu Lys Ser Leu Ala Tyr Phe Met
               260                 265                 270
   Val Ala Pro Thr Leu Cys Tyr Gln Pro Ser Tyr Pro Arg Ser Pro Cys
           275                 280                 285
   Ile Arg Lys Gly Trp Val Ala Arg Gln Leu Ala Lys Leu Val Ile Phe
       290                 295                 300
   Thr Gly Leu Met Gly Phe Ile Ile Glu Gln Tyr Ile Asn Pro Ile Val
   305                 310                 315                 320
   Arg Asn Ser Lys His Pro Leu Lys Gly Asp Leu Leu Tyr Ala Ile Glu
                   325                 330                 335
   Arg Val Leu Lys Leu Ser Val Pro Asn Leu Tyr Val Trp Leu Cys Met
               340                 345                 350
   Phe Tyr Cys Phe Phe His Leu Trp Leu Asn Ile Leu Ala Glu Leu Leu
           355                 360                 365
   Cys Phe Gly Asp Arg Glu Phe Tyr Lys Asp Trp Trp Asn Ala Lys Ser
       370                 375                 380
   Val Gly Asp Tyr Trp Arg Met Trp Asn Met Pro Val His Lys Trp Met
   385                 390                 395                 400
   Val Arg His Val Tyr Phe Pro Cys Leu Arg Ile Lys Ile Pro Lys Val
                   405                 410                 415
   Pro Ala Ile Ile Ile Ala Phe Leu Val Ser Ala Val Phe His Glu Leu
               420                 425                 430
   Cys Ile Ala Val Pro Cys Arg Leu Phe Asn Leu Trp Ala Phe Met Gly
           435                 440                 445
   Ile Met Phe Gln Val Pro Leu Val Phe Ile Thr Asn Phe Leu Gln Glu
       450                 455                 460
   Arg Phe Gly Ser Met Val Gly Asn Met Ile Phe Gly Ser Ala Ser Cys
   465                 470                 475                 480
   Ile Phe Gly Gln Pro Met Cys Gly Leu Leu Tyr Tyr His Asp Leu Met
                   485                 490                 495
   Asn Arg Lys Gly Ser Met Ser
               500
<210> 10
   <211> 11
   <212> PRT
   <213> Arabidopsis thaliana
<220>
   <221> VARIANT
   <222> (1)
<220>
   <221> VARIANT
   <222> (3)
<220>
   <221> VARIANT
   <222> (5)..(6)
<220>
   <221> VARIANT
   <222> (8)..(10)
<400> 10
   Xaa Leu Xaa Lys Xaa Xaa Ser Xaa Xaa Xaa Val
   1               5                   10
<210> 11
   <211> 27
   <212> PRT
   <213> Arabidopsis thaliana
<400> 11
   Ser His Ala Gly Leu Phe Asn Leu Cys Val Val Val Leu Ile Ala Val
   1               5                   10                  15
   Asn Ser Arg Leu Ile Ile Glu Asn Leu Met Lys
               20                  25
<210> 12
   <211> 24
   <212> DNA
   <213> Artificial Sequence
<220>
   <223> Description of Artificial Sequence: Primer of DGATXbal
<400> 12
   ctagtctaga atggcgattt tgga 24
<210> 13
   <211> 23
   <212> DNA
   <213> Artificial Sequence
<220>
   <223> Description of Artificial Sequence: Primer of DGATXhoI
<400> 13
   gcgctcgagt ttcatgacat cga 23
<210> 14
   <211> 24
   <212> DNA
   <213> Artificial Sequence
<220>
   <223> Description of Artificial Sequence: Primer DGAT1
<400> 14
   agacacgaat cccattccca ccga 24
<210> 15
   <211> 24
   <212> DNA
   <213> Artificial Sequence
<220>
   <223> Description of Artificial Sequence: Primer DGAT2
<400> 15
   agtggtgaca acgcagggat gatg 24
<210> 16
   <211> 21
   <212> DNA
   <213> Artificial Sequence
<220>
   <223> Description of Artificial Sequence: Primer DGAT3
<400> 16
   atggtcgctc ccacattgtg t 21
<210> 17
   <211> 25
   <212> DNA
   <213> Artificial Sequence
<220>
   <223> Description of Artificial Sequence: Primer DGAT4
<400> 17
   catacaatcc ccatgacatt tatca 25
<210> 18
   <211> 24
   <212> DNA
   <213> Artificial Sequence
<220>
   <223> Description of Artificial Sequence: Primer A
<400> 18
   cgaccgtcgg ttccagctca tcgg 24
<210> 19
   <211> 24
   <212> DNA
   <213> Artificial Sequence
<220>
   <223> Description of Artificial Sequence: Primer B
<400> 19
   gcggccaatc tcgcagcgat cttg 24
<210> 20
   <211> 21
   <212> DNA
   <213> Artificial Sequence
<220>
   <223> Description of Artificial Sequence: Primer C
<400> 20
   taaacagtag actcatcatc g 21
<210> 21
   <211> 33
   <212> DNA
   <213> Artificial Sequence
<220>
   <223> Description of Artificial Sequence: Primer Gen 1
<400> 21
   gagaggatcc acgctcacga cccattcttc ccg 33
<210> 22
   <211> 33
   <212> DNA
   <213> Artificial Sequence
<220>
   <223> Description of Artificial Sequence: Primer Gen 2
<400> 22
   aagaaggatc catccccaaa acgggaccac caa 33
<210> 23
   <211> 1985
   <212> DNA
   <213> Arabidopsis thaliana
<400> 23
   atttcttagc ttcttccttc aatccgctct ttccctctcc attagattct gtttcctctt 60
   tcaatttctt ctgcatgctt ctcgattctc tctgacgcct cttttctccc gacgctgttt 120
   cgtcaaacgc ttttcgaaat ggcgattttg gattctgctg gcgttactac ggtgacggag 180
   aacggtggcg gagagttcgt cgatcttgat aggcttcgtc gacggaaatc gagatcggat 240
   tcttctaacg gacttcttct ctctggttcc gataataatt ctccttcgga tgatgttgga 300
   gctcccgccg acgttaggga tcggattgat tccgttgtta acgatgacgc tcagggaaca 360
   gccaatttgg ccggagataa taacggtggt ggcgataata acggtggtgg aagaggcggc 420
   ggagaaggaa gaggaaacgc cgatgctacg tttacgtatc gaccgtcggt tccagctcat 480
   cggagggcga gagagagtcc acttagctcc gacgcaatct tcaaacagag ccatgccgga 540
   ttattcaacc tctgtgtagt agttcttatt gctgtaaaca gtagactcat catcgaaaat 600
   cttatgaaga gccatgccgg attattcaac ctctgtgtag tagttcttat tgctgtaaac 660
   agtagactca tcatcgaaaa tcttatgaag tatggttggt tgatcagaac ggatttctgg 720
   tttagttcaa gatcgctgcg agattggccg cttttcatgt gttgtatatc cctttcgatc 780
   tttcctttgg ctgcctttac ggttgagaaa ttggtacttc agaaatacat atcagaacct 840
   gttgtcatct ttcttcatat tattatcacc atgacagagg ttttgtatcc agtttacgtc 900
   accctaaggt gtgattctgc ttttttatca ggtgtcactt tgatgctcct cacttgcatt 960
   gtgtggctaa agttggtttc ttatgctcat actagctatg acataagatc cctagccaat 1020
   gcagctgata aggccaatcc tgaagtctcc tactacgtta gcttgaagag cttggcatat 1080
   ttcatggtcg ctcccacatt gtgttatcag ccaagttatc cacgttctgc atgtatacgg 1140
   aagggttggg tggctcgtca atttgcaaaa ctggtcatat tcaccggatt catgggattt 1200
   ataatagaac aatatataaa tcctattgtc aggaactcaa agcatccttt gaaaggcgat 1260
   cttctatatg ctattgaaag agtgttgaag ctttcagttc caaatttata tgtgtggctc 1320
   tgcatgttct actgcttctt ccacctttgg ttaaacatat tggcagagct tctctgcttc 1380
   ggggatcgtg aattctacaa agattggtgg aatgcaaaaa gtgtgggaga ttactggaga 1440
   atgtggaata tgcctgttca taaatggatg gttcgacata tatacttccc gtgcttgcgc 1500
   agcaagatac caaagacact cgccattatc attgctttcc tagtctctgc agtctttcat 1560
   gagctatgca tcgcagttcc ttgtcgtctc ttcaagctat gggcttttct tgggattatg 1620
   tttcaggtgc ctttggtctt catcacaaac tatctacagg aaaggtttgg ctcaacggtg 1680
   gggaacatga tcttctggtt catcttctgc attttcggac aaccgatgtg tgtgcttctt 1740
   tattaccacg acctgatgaa ccgaaaagga tcgatgtcat gaaacaactg ttcaaaaaat 1800
   gactttcttc aaacatctat ggcctcgttg gatctccgtt gatgttgtgg tggttctgat 1860
   gctaaaacga caaatagtgt tataaccatt gaagaagaaa agaaaattag agttgttgta 1920
   tctgcaaaaa ttttggtaga gacacgcaaa cccgtttgga ttttgttatg gtgtaaagcg 1980
   gccgc                                                             1985
<210> 24
   <211> 5339
   <212> DNA
   <213> Arabidopsis thaliana
<400> 24
   gctcacgacc cattcttccc gttccatttg gttttattta tttcaaagtt taatattcct 60
   tttgtataac attcaaatct tcacatgatt gattgtgtga aaaccccaca gattttacta 120
   caataggggg agttgactta aaatagctat tgatgtcgaa aaaatgtatt ttagttataa 180
   attatactaa agaaaatttt tgatttgtct gttgtttaag catatgtatt gttaaactta 240
   aaaaaatatg tattgttaat cttaaaaatg taggagtaca catcaaatac tcgagcataa 300
   tcaaaaccgt attcatagac cgatgtgaga atcaaataga agataatgtg attttttaaa 360
   atatcgtatc tccaaatcaa tcacttagaa gataatgtaa ttctttatgt gctacataaa 420
   taaatatata tatatatata tatatatatc ttgtatatat gtcttgacaa aaaattgcca 480
   gtcaaaaacc atgactgaat caaactataa gtcggattga atcaaactat aagtcggatg 540
   agtattaatt tccattatgt ttctatactt tacaaaccgg aaaatagata ttatagatac 600
   caaaaaagta gatttgtgta tattattaga agatttggaa tttcatcatt atcaggatct 660
   aaagtacttc cctaattaaa tcatgtcggt tgaaaaagct caatgaatgt ttgaaatttg 720
   gaaagtttat taaattcgga tctttttttt ttgtttgtcg tcccaaacat ttttatttta 780
   ttacaaataa tcaacttatc cttactacta aatcatttca tatctttgat accaacaaat 840
   catttcatat tctattttga tgtttaagaa aacactattt accagttaca aaatattata 900
   aggattgttg tttagaaaaa aaagtacaag ttgaattctt tttgtcaaat ataaaattga 960
   ctttttaata tataattgac ttattgaaca tgattacaga attaatcatc tacaaaactt 1020
   tccaagttta taataaatac atttcaaaga ctattagttc ttcttaaaat atttctaaaa 1080
   gtgatcaaag actaccacat ataattcaga aaaagtagaa gttgatttct ttttgtcaaa 1140
   taaataattg acttaaaata gtttggaaag ccattgaact tgattataga attgataatg 1200
   tacataaaaa aattccaagt ttataataaa tacatttttc aaatgctata tcagttcttc 1260
   ttaaaatatt tcactaaaaa aacactcaaa tatagaataa atttattgaa taacatacca 1320
   actgtaaaac agaatttgac aaaaaaaaaa aaaaaatgaa atgaagatga agacaaaaat 1380
   aaatcaccag aggatcttat gcaaaaaaat atatgaatac acaataaacc atattgatat 1440
   ttttaaaata aaataaaaac agaaaaatat cccaacaccg cttttcaatt aaaaatcttc 1500
   cgtcaccatt gttgtcatct tcctctctcg tgaatccttt ttcctttctt cttcttcttc 1560
   tcttcagaga aaactttgct tctctttcta taaggaacca gacacgaatc ccattcccac 1620
   cgatttctta gcttcttcct tcaatccgct ctttccctct ccattagatt ctgtttcctc 1680
   tttcaatttc ttctgcatgc ttctcgattc tctctgacgc ctcttttctc ccgacgctgt 1740
   ttcgtcaaac gcttttcgaa atggcgattt tggattctgc tggcgttact acggtgacgg 1800
   agaacggtgg cggagagttc gtcgatcttg ataggcttcg tcgacggaaa tcgagatcgg 1860
   attcttctaa cggacttctt ctctctggtt ccgataataa ttctccttcg gatgatgttg 1920
   gagctcccgc cgacgttagg gatcggattg attccgttgt taacgatgac gctcagggaa 1980
   cagccaattt ggccggagat aataacggtg gtggcgataa taacggtggt ggaagaggcg 2040
   gcggagaagg aagaggaaac gccgatgcta cgtttacgta tcgaccgtcg gttccagctc 2100
   atcggagggc gagagagagt ccacttagct ccgacgcaat cttcaaacag gtttaaaatc 2160
   tcagaaatct tcgaatttgg tgtttgcttg ttgttttata tggaattgag tttggtgatt 2220
   gttttgcatt gcagagccat gccggattat tcaacctctg tgtagtagtt cttattgctg 2280
   taaacagtag actcatcatc gaaaatctta tgaaggtttg ctgttacttg tttctccttt 2340
   taggaattga attgcttgaa aatttatcat tgcattgcag agccatgccg gattattcaa 2400
   cctctgtgta gtagttctta ttgctgtaaa cagtagactc atcatcgaaa atcttatgaa 2460
   ggtttgctgt tacttgtttc tccttttagg aattgaattg cttgaaaatt tatcagagac 2520
   gaataacttt gttgttgcta tcattcatgt agtatggttg gttgatcaga acggatttct 2580
   ggtttagttc aagatcgctg cgagattggc cgcttttcat gtgttggtaa aagaagatgt 2640
   tttttatttc cagcaatgtt acattgttat acgtataatg atgagtttag tgatcaagtt 2700
   cctctttgat tcttctttct tgttgcagta tatccctttc gatctttcct ttggctgcct 2760
   ttacggttga gaaattggta cttcagaaat acatatcaga acctgtgagt aattactatt 2820
   ctccagccat tactgtaatt tttattgaag acaagtttgt atcatgaaga acttacaagt 2880
   tctgttttga aaatgctcaa ggttgtcatc tttcttcata ttattatcac catgacagag 2940
   gttttgtatc cagtttacgt caccctaagg tgatactgtt tttctggtct cagtttgtga 3000
   tactgttttt aagtttagtt gtctgacccg gtgatcttga aaatggacag gtgtgattct 3060
   gcttttttat caggtgtcac tttgatgctc ctcacttgca ttgtgtggct aaagttggtt 3120
   tcttatgctc atactagcta tgacataaga tccctagcca atgcagctga taaggtaaaa 3180
   tacgaaaaag aagcgtatgt attagtcact tgcactgtgt tactgtttta accaaacact 3240
   gttatgaact ttaggccaat cctgaagtct cctactacgt tagcttgaag agcttggcat 3300
   atttcatggt cgctcccaca ttgtgttatc aggtaactgc aaagtgcatc aaccattctt 3360
   atacttgcaa gagtttcttg tctaaacctc ggatctttgc ttttccccag ccaagttatc 3420
   cacgttctgc atgtatacgg aagggttggg tggctcgtca atttgcaaaa ctggtcatat 3480
   tcaccggatt catgggattt ataatagaac aagtacgttt tcacatcttg ctttattagt 3540
   tttccttggt gaaaatcatc atccctgcgt tgtcaccact tgacttcatg ttcttttgtt 3600
   acattttggc agtatataaa tcctattgtc aggaactcaa agcatccttt gaaaggcgat 3660
   cttctatatg ctattgaaag agtgttgaag ctttcagttc caaatttata tgtgtggctc 3720
   tgcatgttct actgcttctt ccacctttgg tatgctgtga tcccatctct ttcaaaataa 3780
   tttgcaaatt cgaaaaaccg aaaaaggcta aatctcatac gaatttgata tttttagttt 3840
   cttagagtcg gtgatgtaat ttcagttact gaacgcaaat ctcttgtcca aaggttaaac 3900
   atattggcag agcttctctg cttcggggat cgtgaattct acaaagattg gtggaatgca 3960
   aaaagtgtgg gagatgtgag ctattttact caaaagaaaa cttatgattt ttaatgttgt 4020
   cgttgttttt gggtcatcta actaaccaaa ttcatgtatt cactgtcttc ctttatcagt 4080
   actggagaat gtggaatatg gtatggttct cttcctaaac atcaccttct tttgtacaca 4140
   aaatagaaga agagagctaa ttaagatctt gttttccttg acagcctgtt cataaatgga 4200
   tggttcgaca tatatacttc ccgtgcttgc gcagcaagat accaaaggtg agtgagatat 4260
   ataccgatat gcaattgtcg agatttgttt ctgtgatata aatttaaccc tccacacact 4320
   tgtttttcag acactcgcca ttatcattgc tttcctagtc tctgcagtct ttcatgaggt 4380
   atacatactt tctacattgc cctgtctcta gacgcatgaa cacacgctag tgaaagaaat 4440
   gctaatattc aaagcattgt ttttacttaa cgatcttgtg ttacaaattt ccttttgaca 4500
   gctatgcatc gcagttcctt gtcgtctctt caagctatgg gcttttcttg ggattatgtt 4560
   tcaggttaaa aaattactaa actgctgcag tcgattttta ctaaactcta atctcatatt 4620
   ctgaccaacc aatttgtttg agtaggtgcc tttggtcttc atcacaaact atctacagga 4680
   aaggtttggc tcaacggtat gctctcaaaa cccgagaaaa tagaacgaat aactctttct 4740
   ttcatagcct agccatttaa atcgcaatgc tgaaacttaa taataaaggt gatctgtttt 4800
   ggaatgggat catattatta ggtggggaac atgatcttct ggttcatctt ctgcattttc 4860
   ggacaaccga tgtgtgtgct tctttattac cacgacctga tgaaccgaaa aggatcgatg 4920
   tcatgaaaca actgttcaaa aaatgacttt cttcaaacat ctatggcctc gttggatctc 4980
   cgttgatgtt gtggtggttc tgatgctaaa acgacaaata gtgttataac cattgaagaa 5040
   gaaaagaaaa ttagagttgt tgtatctgca aaaattttgg tagagacacg cgaacccgtt 5100
   tggattttgt tatggtgtaa agaaatttca atcaaaaaac tgttgtaata attgttacca 5160
   aaaagaaatg cttttctgga aacgagggga aaaatagtag ttttgttagg ttttactgtt 5220
   tggaccaaat ctagtaaaaa actttttgta ataaggaaaa aaaaagaaca aatgtgataa 5280
   atgcatgggg attgtatgaa accttccaat aaagttgatt ggtggtcccg ttttgggga  5339
<210> 25
   <211> 547
   <212> PRT
   <213> Arabidopsis thaliana
<400> 25
   Met Ala Ile Leu Asp Ser Ala Gly Val Thr Thr Val Thr Glu Asn Gly
   1               5                   10                  15
   Gly Gly Glu Phe Val Asp Leu Asp Arg Leu Arg Arg Arg Lys Ser Arg
               20                  25                  30
   Ser Asp Ser Ser Asn Gly Leu Leu Leu Ser Gly Ser Asp Asn Asn Ser
           35                  40                  45
   Pro Ser Asp Asp Val Gly Ala Pro Ala Asp Val Arg Asp Arg Ile Asp
       50                  55                  60
   Ser Val Val Asn Asp Asp Ala Gln Gly Thr Ala Asn Leu Ala Gly Asp
   65                  70                  75                  80
   Asn Asn Gly Gly Gly Asp Asn Asn Gly Gly Gly Arg Gly Gly Gly Glu
   85 90 95
   Gly Arg Gly Asn Ala Asp Ala Thr Phe Thr Tyr Arg Pro Ser Val Pro
   100 105 110
   Ala His Arg Arg Ala Arg Glu Ser Pro Leu Ser Ser Asp Ala Ile Phe
   115 120 125
   Lys Gln Ser His Ala Gly Leu Phe Asn Leu Cys Val Val Val Leu Ile
   130 135 140
   Ala Val Asn Ser Arg Leu Ile Ile Glu Asn Leu Met Lys Ser His Ala
   145 150 155 160
   Gly Leu Phe Asn Leu Cys Val Val Val Leu Ile Ala Val Asn Ser Arg
   165 170 175
   Leu Ile Ile Glu Asn Leu Met Lys Tyr Gly Trp Leu Ile Arg Thr Asp
   180 185 190
   Phe Trp Phe Ser Ser Arg Ser Leu Arg Asp Trp Pro Leu Phe Met Cys
   195 200 205
   Cys Ile Ser Leu Ser Ile Phe Pro Leu Ala Ala Phe Thr Val Glu Lys
   210 215 220
   Leu Val Leu Gln Lys Tyr Ile Ser Glu Pro Val Val Ile Phe Leu His
   225 230 235 240
   Ile Ile Ile Thr Met Thr Glu Val Leu Tyr Pro Val Tyr Val Thr Leu
   245 250 255
   Arg Cys Asp Ser Ala Phe Leu Ser Gly Val Thr Leu Met Leu Leu Thr
   260 265 270
   Cys Ile Val Trp Leu Lys Leu Val Ser Tyr Ala His Thr Ser Tyr Asp
   275 280 285
   Ile Arg Ser Leu Ala Asn Ala Ala Asp Lys Ala Asn Pro Glu Val Ser
   290 295 300
   Tyr Tyr Val Ser Leu Lys Ser Leu Ala Tyr Phe Met Val Ala Pro Thr
   305 310 315 320
   Leu Cys Tyr Gln Pro Ser Tyr Pro Arg Ser Ala Cys Ile Arg Lys Gly
   325 330 335
   Trp Val Ala Arg Gln Phe Ala Lys Leu Val Ile Phe Thr Gly Phe Met
   340 345 350
   Gly Phe Ile Ile Glu Gln Tyr Ile Asn Pro Ile Val Arg Asn Ser Lys
   355 360 365
   His Pro Leu Lys Gly Asp Leu Leu Tyr Ala Ile Glu Arg Val Leu Lys
   370 375 380
   Leu Ser Val Pro Asn Leu Tyr Val Trp Leu Cys Met Phe Tyr Cys Phe
   385                 390                 395                 400
   Phe His Leu Trp Leu Asn Ile Leu Ala Glu Leu Leu Cys Phe Gly Asp
                   405                 410                 415
   Arg Glu Phe Tyr Lys Asp Trp Trp Asn Ala Lys Ser Val Gly Asp Tyr
               420                 425                 430
   Trp Arg Met Trp Asn Met Pro Val His Lys Trp Met Val Arg His Ile
           435                 440                 445
   Tyr Phe Pro Cys Leu Arg Ser Lys Ile Pro Lys Thr Leu Ala Ile Ile
       450                 455                 460
   Ile Ala Phe Leu Val Ser Ala Val Phe His Glu Leu Cys Ile Ala Val
   465                 470                 475                 480
   Pro Cys Arg Leu Phe Lys Leu Trp Ala Phe Leu Gly Ile Met Phe Gln
                   485                 490                 495
   Val Pro Leu Val Phe Ile Thr Asn Tyr Leu Gln Glu Arg Phe Gly Ser
               500                 505                 510
   Thr Val Gly Asn Met Ile Phe Trp Phe Ile Phe Cys Ile Phe Gly Gln
           515                 520                 525
   Pro Met Cys Val Leu Leu Tyr Tyr His Asp Leu Met Asn Arg Lys Gly
       530                 535                 540
   Ser Met Ser
   545

## Description

### TECHNICAL FIELD

This invention relates to plant genes useful for the genetic manipulation of plant characteristics. More specifically, the invention relates to the identification, isolation and introduction of diacylglycerol acyltransferase (DGAT) genes useful, for example, for altering the seed oil content, the ratio of diacylglycerol / triacylglycerol proportions in the seed oil, fatty acid synthesis, seed oil acyl composition, seed size/weight and carbon flux into other seed components, in commercial or crop plants.

### BACKGROUND ART

Plant seed oils are major sources of essential polyunsaturated fatty acids for human diets and renewable feedstocks for chemical industries. The enzymes of the fatty acid synthase complex in the plastids of developing seeds are responsible for the biosynthesis of fatty acids that are channeled into the cytosolic acyl-CoA pool to sustain triacylglycerol accumulation. Triacylglycerol (TAG) biosynthesis is located in the endoplasmic reticulum with glycerol-3-phosphate and fatty acyl-CoAs as the primary substrates. There are three acyltransferases involved in the plant storage lipid bioassembly, namely the glycerol-3-phosphate acyltransferase (GPAT, EC 2.3.1.15), the *lyso*-phosphatidic acid acyltransferase (LPAT, EC 2.3.1.51) and the diacylglycerol acyltransferase (DGAT, EC 2.3.1.20). These three acyltransferases catalyze the stepwise acylation of the glycerol backbone with the final step being the acylation of sn-1, 2-diacylglycerol (DAG) by DGAT into the formation of TAGs, a biochemical process generally known as the Kennedy pathway (Stymne and Stobart, 1987).

Among the three ER-based fatty acyl-CoA acyltransferases, only LPAT gene(s) have been cloned from plants (Knutzon *et al.,* 1995, Lassner et *al.,* 1995). Like several other enzymes involved in storage lipid biosynthesis, acyltransferases are intrinsic ER proteins and are extremely difficult to purify. The research on plant DGAT has been largely limited to studies of activity profiles by using the particulate fractions generated by differential centrifugation of seed or microspore-derived embryo homogenates (Weselake et al., 1993). Although partial purification of DGAT from cotyledons of germinating soybean seeds was reported (Kwanyuan and Wilson, 1986), detailed molecular characterization of this enzyme is lacking.

Reference is made to R61u012 Database Entry Ac005917; Accession number AC005917; 4 November 1998; LIN X. ET AL : "Arabidopsis thaliana chromosome II section 113 of 255 of the complete sequence". This relates to a nucleotide sequence deposit first submitted to the NCBI GenBank on November 3, 1998 without any identification of putative coding sequences contained therein. There was no reference in the deposited materials to a diacylglycerol O-acyltransferase gene".

Reference is also made to VESNA KATAVlC ET AL.: "Alteration of Seed Fatty Acid Composition by an Ethyl Methanesulfonate-induced Mutation in Arabidopisis thaliana Affecting Diacylglycerol Acyltransferase Activity": PLANT PHYSIOLOGY, vol. 108, 1995, pp. 399-409. This reference discloses an Arabidopsis mutant designated AS11 that has reduced diacylglycerol acyltransferase activity. The reference does not disclose any DNA sequences whatsoever and teaches only that alterations in DGAT activity may lead to changes In fatty acid content.

Accordingly, while the Kennedy pathway is known and shows the steps in the biosynthesis of TAGs in plants, there has not been any identification and use of a genetic element that can be used reliably in plants to modify TAG synthesis and composition in a way that may be exploited commercially.

### DISCLOSURE OF THE INVENTION

An object of the invention is to identify, isolate and clone a genetic element that may be used to modify the natural formation of triacylglycerols in plants in order to increase the yield of commercial plant oils, or to modify their composition to achieve specific commercial improvements of plants and plant products.

Another object of the invention is to identify, isolate and characterize diacylglycerol acyltransferase (DGAT) gene and cDNA sequences from Arabidopsis and to utilize these sequences in the genetic manipulation of plants.

Another object of the invention is to provide a vector containing the full-length DGAT cDNA sequence from *Arabidopsis* in a sense orientation under the control of a seed-specific promoter (e.g. napin; See Josefsson et al., 1987; Radke et al., 1988; Voelker et al., 1992), for re-introducing into *Arabidopsis* or for introducing into other plants.

Another object of the invention is to provide a vector containing a genomic fragment from *Arabdopsis* consisting of the full-length DGAT gene under the control of its own 5' upstream regulatory sequences, for re-introducing into *Arabidopsis* or for introducing into other plants.

Another object of the invention is to provide a method to construct a vector containing the full-length DGAT sequence or a significant portion of the DGAT sequence from *Arabidopsis,* in an antisense orientation under control of either a constitutive or a seed-specific promoter, for re-introducing into *Arabidopsis* or for introducing into other plants.

Another object of the invention is to provide a method of modifiying *Arabidopsis* and other plants to change their seed oil content.

Another object of the invention is to provide a method of modifiying *Arabidopsis* and other plants to change the acyl composition of their seed oil.

Another object of the invention is to provide a method of modifiying *Arabidopsis* and other plants to change their average seed weight or size.

According to one aspect of the present invention, there is provided a vector containing isolated and purified deoxyribonucleic acid (cDNA) of SEQ ID NO:1 (pDGATcDNA; ATCC No PTA-989), for introduction of the cDNA in a sense orientation into a plant cell.

According to yet another object of the invention, there is provided a method for preparing a vector containing SEQ ID NO:1 or a part thereof, for introduction of the gene or partial gene in an antisense orientation, into a plant cell.

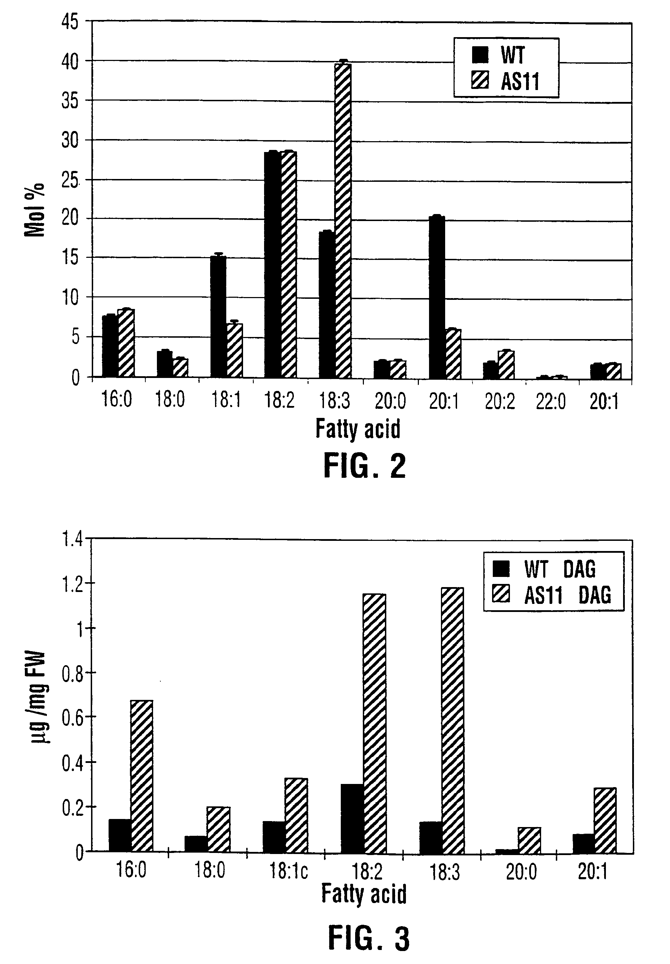

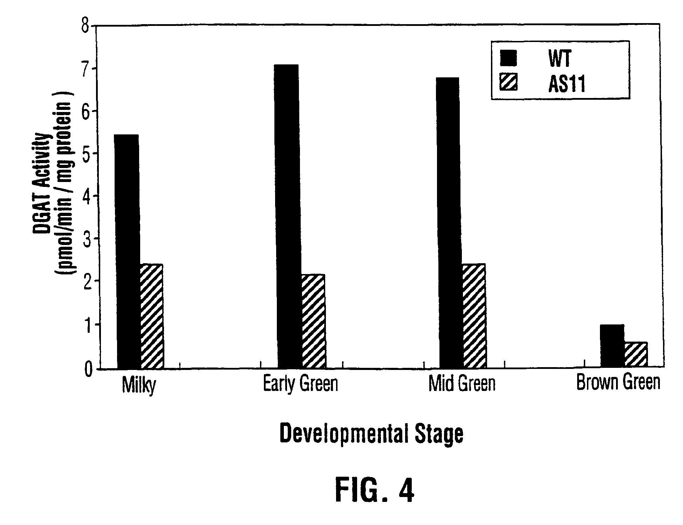

According to yet another object of the invention, there is provided seed of *Arabidopsis thaliana* ecotype Columbia mutant AS11 (ATCC No. PTA-1013) and characterization of its lipid phenotype (Katavic et al., 1995; Zou et al. 1999). The AS11 mutant seed line has an insertion mutation at the *TAG1* locus on chromosome 11, and produces plants exhibiting reduced DGAT activity (Figure 4) and an reduced TAG/DAG ratio during seed development (Table 1), resulting in an altered seed fatty acyl composition (Figure 2), reduced oil content (Table 1), and increased seed oil diacylglycerol content during development (Figure 3) and at maturity (lower TAG/DAG ratio cf Table 1). The cDNA sequence of the AS11 DGAT is shown in SEQ ID NO:23, the genomic DNA sequence is shown in SEQ ID NO:24 and the translated protein sequence of the AS11 DGAT is shown in SEQ ID NO:25.

The invention also relates to transgenic plants and plant seeds having a genome containing an introduced DNA sequence of SEQ ID NO: 1 and a method of producing such plants and plant seeds.

The invention also relates to SEQ ID NO:1, or a part of SEQ ID NO:1, or SEQ ID NO:1 containing an 81 bp insertion [SEQ ID NO: 23] or SEQ ID NO:3 containing an 147 bp insertion [SEQ ID NO:24] such that the deduced amino acid sequence of the encoded protein contains the repeated sequence SHAGLFNLCVVVLIAVNSRLIIENLMK [SEQ ID NO:25] where the spacing and identity of the underlined amino acids are identical or are replaced by conserved substitutions, characterized in that said sequence has been introduced in sense or antisense orientation, and a method of producing such plants and plant seeds.

Stated more generally, the present invention relates to the isolation, purification and characterization of a diacylglycerol acyltransferase (DGAT) gene from the *Brassicaceae* (specifically *Arabidopsis thaliana)* and demonstrates its utility in regulating fatty acid synthesis, seed oil content, diacylglycerol/triacylglycerol ratios and seed size/weight. Until now, no concrete data is available on the gene structure of plant DGATs, or their utility in altering oil content or composition through genetic manipulation.

When considering altered oil contents or compositions, results from averages of statistically-significant numbers of plants or seeds according to the invention are best compared with results from averages of statistically-significant numbers of untransformed (control) plants or seeds of the same genotype grown under identical conditions at the same time. This allows for the variability of individual plants of the same genotype, particularly when such plants are grown under different conditions. The actual number of plants or seeds used to form the required average may vary, but should be enough to provide a generally constant average whenever such number is selected. Generally, the number should be at least 10, and is more preferably at least 20, 30, 50 or 100.

The DGAT gene was cloned, characterized and authenticated from *Arabidopsis* by: (1) selection and characterization of plant ESTs sharing some homology to mammalian acyl-CoA: cholesterol acyltransferases; (2) the functional expression of a full-length cDNA in yeast; (3) the characterization and isolation of the DGAT *(TAG1)* gene from Arabidopsis mutant AS11 containing an insertion mutation In the DGAT gene and a seed oil phenotype which consists of an altered DAG/TAG ratio, and an altered oil content and acyl composition; (4) complementation of the AS11 mutant by insertion of the DGAT cDNA sequence to restore a wild-type fatty acid composition: (5) the over-expression of the DGAT cDNA in wild-type *A. thaliana* transgenic plants which produce seeds with an increased oil content, increased average seed weight and altered seed oil acyl composition.

The *A. thaliana* DGAT structure is significantly homologous (over 40% identical over a region of more than 400 amino acids) to its mammalian counterparts, and is highly homologous to subsequently reported putative *B. napus* DGATs at both the nucleotide (92%) and the deduced amino acid (90%) levels (Nykiforuk et al, 1999; GenBank/EMBL Accession No AF155224; AF164434).

The DGAT of the current invention is useful in manipulating DGAT activity, and triacylglycerol bioassembly in plants. For example, by transforming plants with a construct containing the DGAT gene in a sense orientation, under the control of a tissue-specific promoter (e.g. seed-specific promoter napin), the expression of DGAT and accumulation of seed oil can be enhanced or the acyl composition of the seed oil altered. Yet another example would be to express the DGAT cDNA under the control of a constitutive promoter (e.g. 35S (Datia et al., 1993)) to Increase the TAG content of vegetative tissues (leaves, roots, stems). This may have particular advantages for altering the starch/oil ratio in root crops.

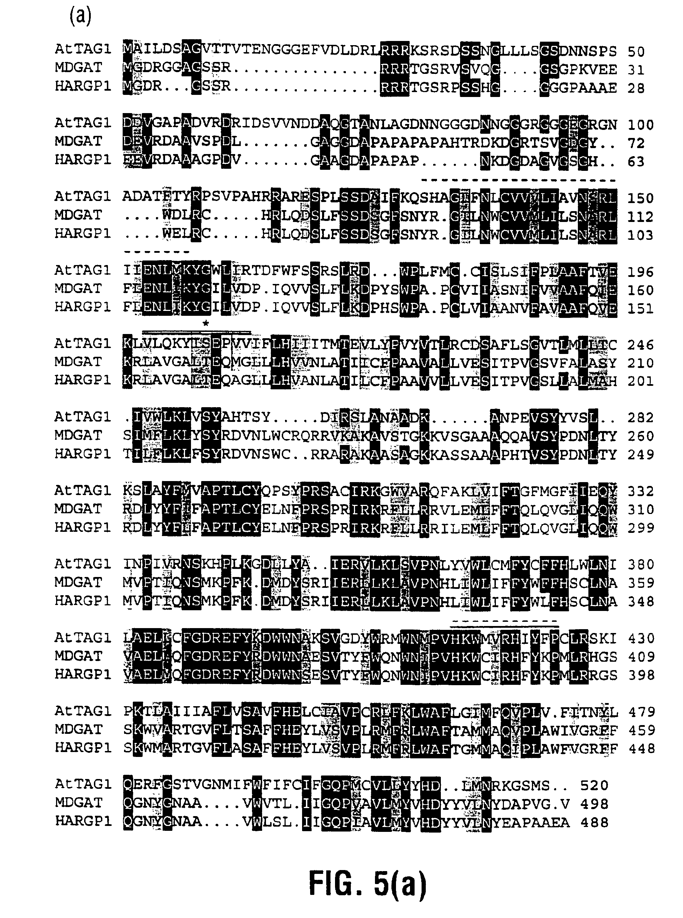

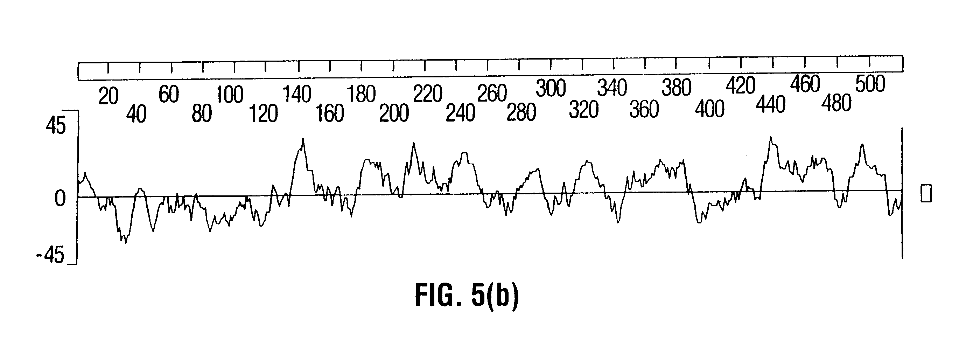

Alternatively, DGAT expression can be silenced to some degree by anti-sense or co-suppression (Transwitch) phenomena (De Lange et al., 1995; Mol et al., 1990; Jorgensen and Napoli, 1994; Kinney, 1995; Vaucheret et al, 1998; Taylor, 1998). For example, silencing DGAT in a seed specific manner may result in a reduction in TAG accumulation. This could have applications in reducing the oil content in seed barley to enhance stability during storage. As a second example, seed-specific silencing may lead to a relatively high accumulation of DAG or an increase in the DAG/TAG ratio in the developing or mature seed. As yet another example, the expression of the mutated DGAT gene which results in a 27 amino acid repeat insertion in the mutant DGAT protein (See Figure 5 a) can be used to alter the DAG/TAG ratio in developing and mature seed. Such manipulations can lead to edible seed oils produced naturally in the plant, containing enhanced relative levels of DAG /reduced levels of TAG (See Figure 3; Table 1) to act as all-natural emulsifiers in the food and confections industries, or to enhance the nutritional/health profile of vegetable oils as functional foods (e.g. as cooking oils, stir fry oils, in salad dressings, margarines etc.) by inhibiting neutral fat deposition in humans. Processed oils produced from canola and soybean which contain increased proportions of diacylglycerol have been cited by the Kao Corporation of Japan (e.g. Econa Cooking Oil; Kao Corporation Kl, 1-14-10 Nihonbashi-Kayabacho, Chuoku, Tokyo 103 Japan; e-mail: 210064@kastanetkao.co.jp) as a product making it difficult for blood neutral fat to increase after a meal, and for fat to cling to the body, thereby assisting individuals who are overweight or who suffer from high neutral fat levels. As a third example, silencing or reducing the activity of DGAT in a seed specific manner (as observed in mutant AS11; e.g. by over-expressing SEQ ID NO:23 or silencing expression of SEQ ID NO:1, and combining this trait with the capacity to produce polyhydroxyalkanoates (PHAs; e.g. polyhydroxybutyrate) in seeds (Poirier et al., 1992; 1995) will allow an increased flow of unesterified fatty acids towards β-oxidation (Poirier et al., 1999). By recycling or diverting the unesterified fatty acids into β oxidation, the resulting acetyl-CoA moieties will lead to a significant increase in polyhydroxyalkanoates (PHAs) or a change in PHA composition (Poirier et al., 1999). Transgenic plants producing PHAs in seeds have potential for utility as biodegradeable thermoplastics. However, up to now, the levels of PHAs produced have been relatively small (Poirier et al, 1992; 1995). The utility of transgenically reducing the DGAT activity to significantly enhance PHA production (e.g. 10-fold increase) in PHA-producing seeds is now possible, due to the current DGAT invention.

Some of the manipulations and deliverables which are possible using the DGAT gene or a part therof, include, but are not limited to, the following: seeds with increased or decreased oil content; seeds containing oils with an enhanced diacylglycerol content, seed oils with an altered acyl composition; plants producing larger or heavier seeds; plants exhibiting an enhanced or altered capacity to accumulate storage compounds in other storage organs (e.g. tubers, roots).

### BRIEF DESCRIPTION OF THE DRAWINGS

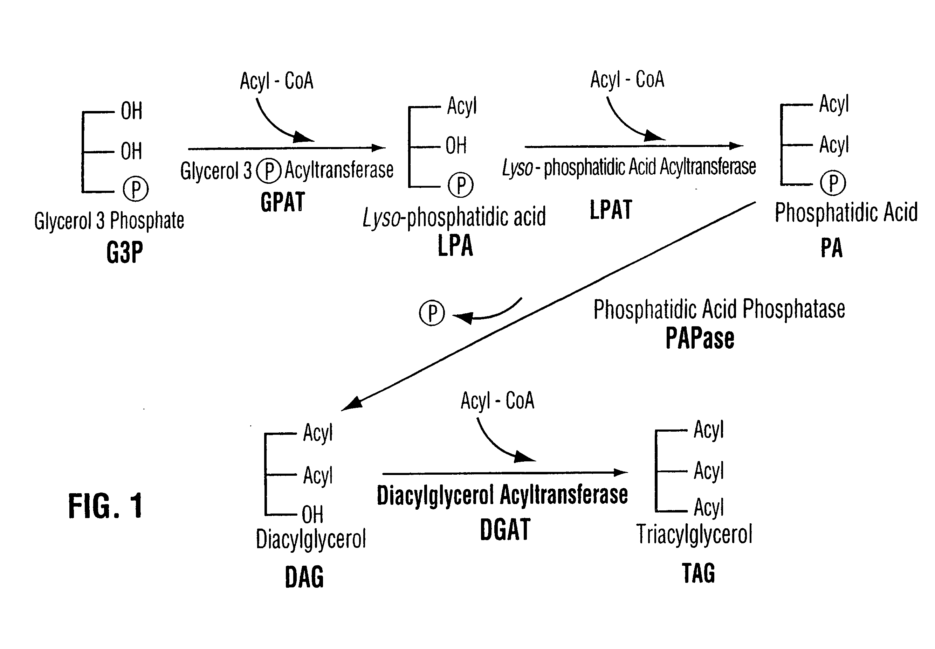

Figure 1 is a diagram illustrating the Kennedy pathway for the bioassembly of triacylglycerols in plants, and shows the critical role played by DGAT as the final step of the Kennedy pathway.
Figure 2 is a graph showing the comparison of the fatty acid composition of seed oil from wild-type (WT) and DGAT mutant AS11 lines of *Arabidopsis thaliana.* Proportions of fatty acids are reported as Mol % of the total fatty acid composition of the seed oil from each line. Error bars are ± SE (n= 10 plants of each line sampled, with 50 seeds per sample per analysis).
Figure 3 is a graph showing a comparison of the DAG content in developing seed of wild-type (WT) and DGAT mutant AS11 lines of *Arabidopsis thaliana.* More specifically, it is a comparison of the fatty acid content of DAG pool in wild-type green developing seeds compared to that of the AS11 mutant.
Figure 4 is a graph showing a comparison of the DGAT activity (pmol/min/mg protein) in developing seeds at the milky, early green, mid-green and brown-green stages of embryo development in wild-type (WT) and DGAT mutant AS11 lines of *Arabidopsis thaliana.* Developing seeds at each stage were selected and DGAT enzyme analyses conducted as described previously by Katavic et al., 1995).
Figure 5(a) shows the amino acid sequence alignment of the *Arabidopsis* DGAT (AtTAG1) [SEQ ID NO:2] with mammalian (mouse and putative human) DGATs. MDGAT, mouse DGAT [SEQ ID NO:4]; GenBank/EMBL Accession No. AF078752 (Cases et al., 1998)]; HARGP1, human ARGP1 protein (SEQ ID NO:5]; GenBank/EMBL Accession No. AF059202; Oelkers et al., 1998]. Dots indicate gaps. Identical amino acid residues are highlighted in black. Conserved residues are shaded. The 27-amino acid repeat found in *Arabidopsis thaliana* mutant AS11 and generated by the insertion mutation (81 bp) found in SEQ ID NO:23 (SHAGLFNLCVVVLIAVNSRLIIENLMK) is overlined thus: --------------. The putative diacylglycerol binding site is overlined thus: 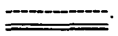 The SnRK1 targeting site is overlined thus: 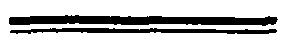 with an asterisk (*) over the serine (S) residue as the targeted phosphorylation site.
Figure 5(b) shows the Kyte-Doolittle hydropathy plot of the DGAT protein.

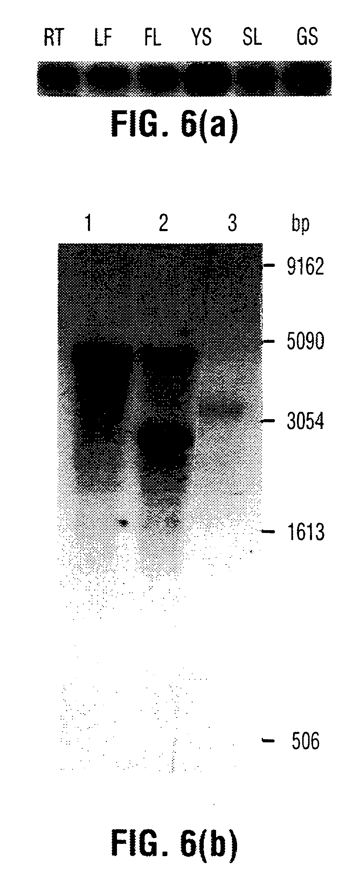

Figure 6(a) shows the results of a Northern analysis of *TAG1* gene expression in *Arabidopsis thaliana.* Total RNA was extracted from roots (RT), leaves (LF), flowers (FL), young seedlings (YS), developing siliques (SL), and germinating seeds (GS).
Figure 6(b) shows the results of Southern blot analysis of the *TAG1* gene in *Arabidopsis thaliana.* Genomic DNA was digested with restriction enzymes BgIII (Lane 1), EcoRI (Lane 2), and HindIII (Lane 3). The *TAG1* DNA probe was ³²P labeled by random priming.

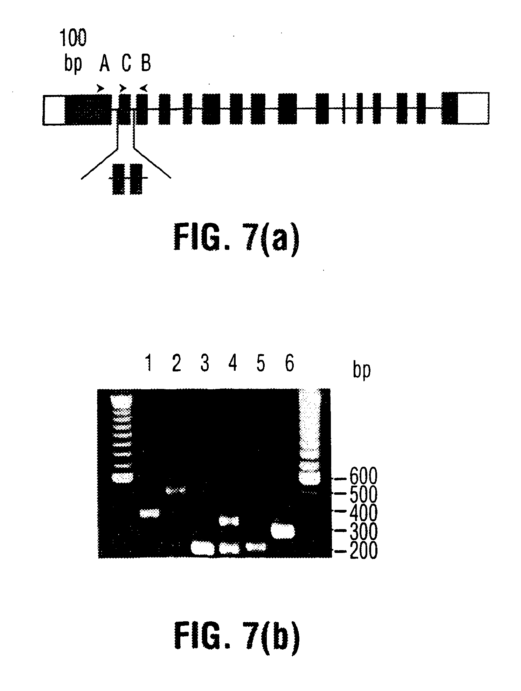

Figure 7(a) is a diagrammatic representation of the *TAG1* gene structure. The boxes indicate the 16 exons (solid boxes for coding regions, open box for untranslated regions), and the lines represent the 15 introns. A, B and C denote the positions of the primers used for PCR amplifications of the segments from wild type (WT) and AS11. The specific primers A, B and C are described in Experimental Procedures: *Primer Strategy* (found later in this specification).
Fig 7(b) shows gel separation of the PCR products amplified from wild type (WT) and AS11. Lane 1, PCR product with primers A and B using WT genomic DNA as template. Lane 2, PCR product with primers A and B using AS11genomic DNA as template. Lane 3, PCR product with primers C and B using WT genomic DNA as template. Lane 4, PCR product with primers C and B using AS11 genomic DNA as template. Lane 5, RT-PCR with primers A and B using RNA prepared from WT seedling RNA. Lane 6, RT-PCR with primers A and B using RNA prepared from AS11 seedling RNA.

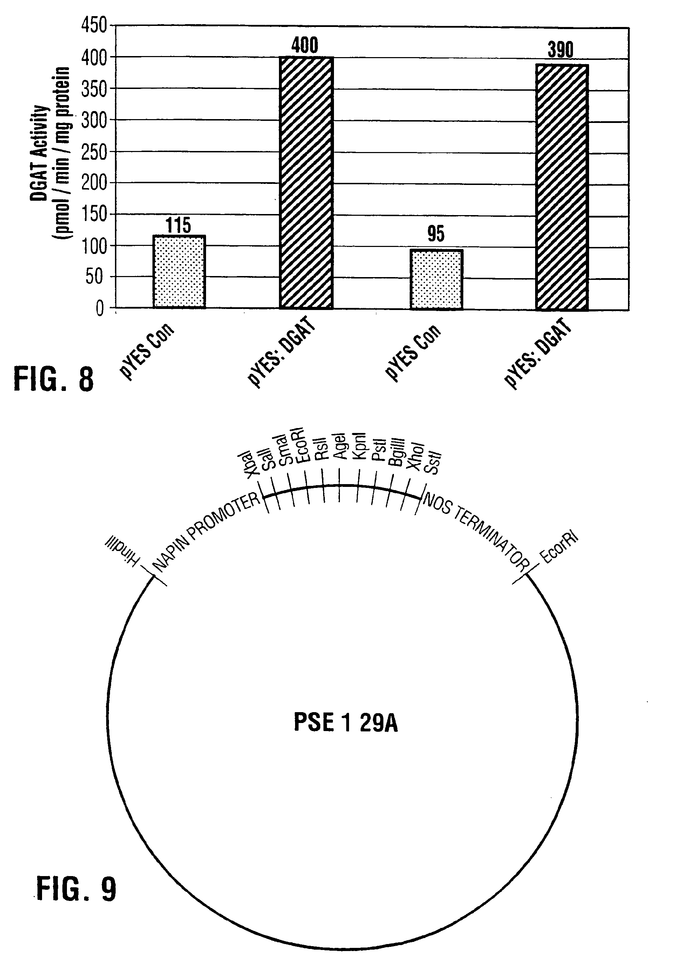

Figure 8 is a graph showing microsomal DGAT activity in Yeast Strain YMN5 Transfomred with empty plasmid (pYES Con) and with the *A*. *thaliana* DGAT cDNA (pYES:DGAT). This illustrates the expression of the *TAG1* cDNA in yeast. Host cultures of strain YMN5 were transformed with *pYES2* plasmid only *(pYES2;* without *TAG1* insert) or with *pYES2* containing the *TAG1* cDNA insert *(pYES2:TAG1).* Following induction in the presence of galactose, transformants were lysed and assayed for DGAT activity as described in the Experimental Procedures. The results of two separate DGAT activity experiments are shown.
Figure 9 is a map of plasmid pSE129A which may be used as a vector. The vector contains the following salient features for plant transformation in the current invention: the seed-specific napin promoter and NOS terminator between which is a multiple cloning site.

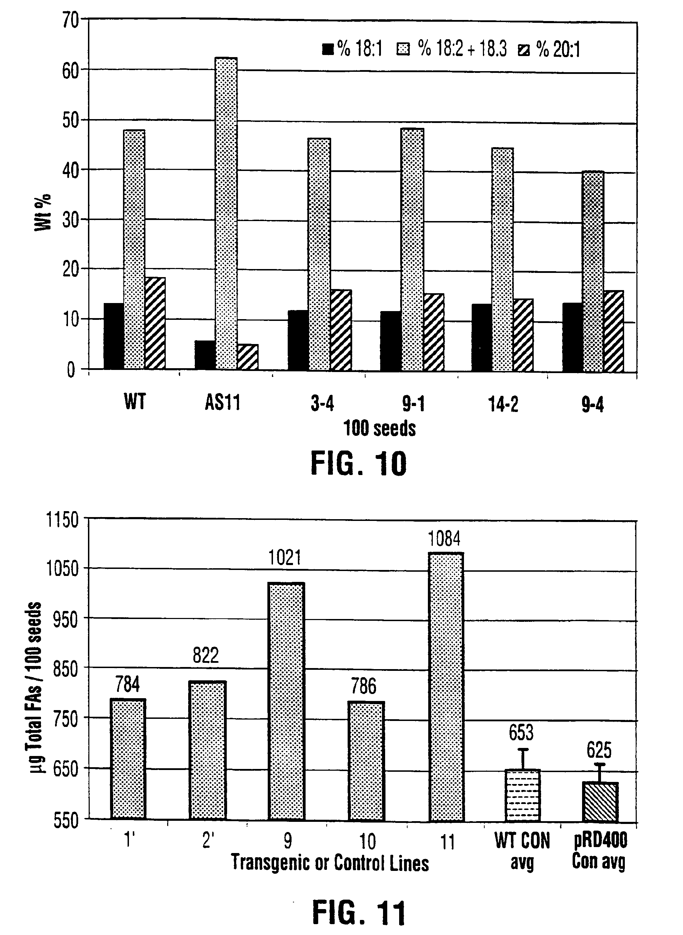

Figure 10 is a graph showing the complementation of the AS11 DGAT mutation with the wild-type cDNA. Transformation of *Arabidopsis thaliana* mutant line AS11 with the DGAT cDNA [SEQ ID NO:1] under the control of a napin promoter, leads to a restoration of the wild-type (WT) fatty acid composition in the seed oil of the transformant lines 3-4, 9-1, 14-2 and 9-4. Fatty acid composition (wt %) was determined on the seed oil extracted from 100-seed samples from A. *thaliana* non-transformed controls (WT), mutant line AS11, and T₂ seeds of napin:DGAT transgenic lines.
Figure 11 is a graph showing the seed oil content of non-transformed WT control, and pRD400 control (empty plasmid) and napin:DGAT T₂ transgenic *Arabidopsis thaliana* seed lines. More particularly, the graph shows the transformation of wild type (WT) *Arabidopsis thaliana* with the DGAT cDNA [SEQ ID NO:1] under the control of a napin promoter, leads to a higher seed oil content in the DGAT transgenic lines. Oil content is expressed as µg total fatty acids (FAs) per 100 seeds from A. *thaliana* non-transformed controls (WT Con), and T₂ seeds of pRD400 control (empty plasmid) transgenic, and napin:DGAT transgenic lines 1', 2', 9, 10 and 11. Standard error bars for the control lines are indicated; n=10.

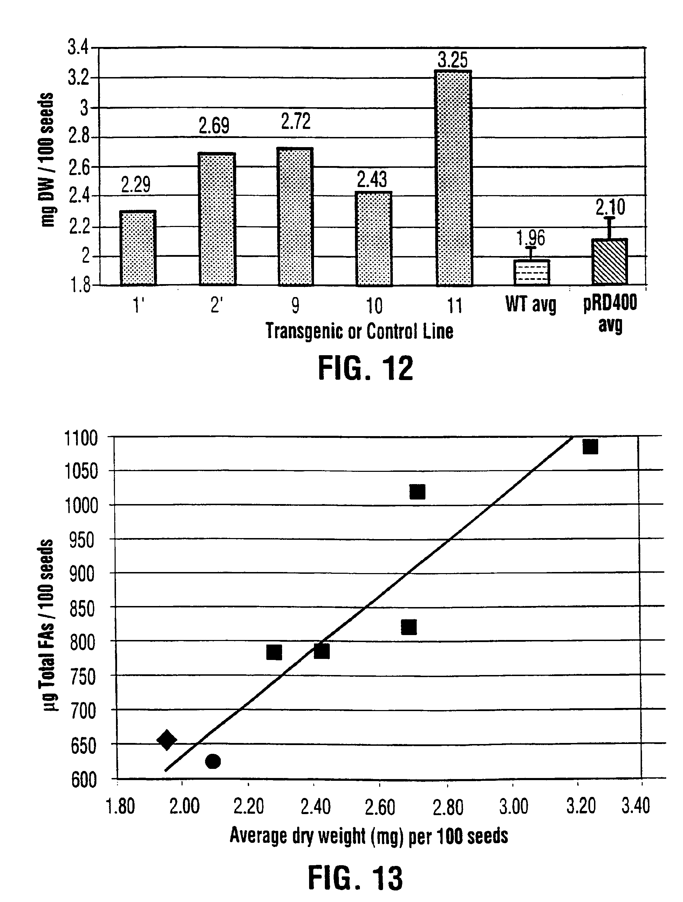

Figure 12 is a graph showing the average 100-seed weight of non-transformed WT control, and pRD400 control (empty plasmid) and napin:DGAT T₂ transgenic *Arabidopsis thaliana* seed lines. More specifically, the graph shows the over-expression of the DGAT cDNA under the control of a napin promoter, in wild-type (WT) *Arabidopsis thaliana* leads to a higher average seed weight. The average weight of 100-seed samples from *A. thaliana* non-transformed controls (WT Con), and T₂ seeds of pRD400 control (empty plasmid) transgenic, and napin:DGAT transgenic lines 1', 2', 9, 10 and 11 are reported as mg dry weight (DW).
Figure 13 is a graph showing the positive correlation between oil content (expressed as pg Total fatty acids (FAs) per 100 seeds) and average seed weight (expressed as average mg DW per 100-seed samples) from A. thaliana non-transformed controls (WT Con ◆), and T2 seeds of pRD400 control (empty plasmid) transgenic (( ) and napin:DGAT transgenic lines 1', 2', 9, 10 and 11 (■) are reported as mg dry weight (DW).

### BEST MODES FOR CARRYING OUT THE INVENTION

Fig. 1 is a diagram illustrating the Kennedy pathway for the biosynthesis of TAGs in plants. Of the various illustrated enzymes, DGAT is the only enzyme in the Kennedy pathway that is exclusively committed to TAG biosynthesis, and its key role is apparent from the scheme of Figure 1. sn-1,2-DAG, generated as a result of either the catalytic action of PA phosphatase (EC 3.1.3.4) or CPTase (EC 2.7.8.2), can be used in the biosynthesis of TAG.

For this reason, the inventors of the present invention decided to investigate DGAT to see if the corresponding gene in plants could be sequenced and cloned and used to modify the seed oil content and fatty acid composition of plants in a way that could be commercially useful.

The acyl-CoA dependent acylation of sn-1,2-DAG is catalyzed by DGAT (Stymne and Stobart, 1987). In developing and germinating seeds of oilseed plants, TAG accumulation and DGAT activity have been shown to associate with the endoplasmic reticulum (ER; high speed microsomal fraction) (Stobart et al., 1986; Cao and Huang, 1986; Stymne and Stobart, 1987; Frentzen, 1993; Settlage et al, 1995; Lacey and Hills, 1996). The biochemical properties of microsomal DGAT have been examined in a number of plant systems (Frentzen, 1993) including developing seeds (Bernerth and Frentzen, 1990; Vogel and Browse, 1996; Cao and Huang, 1987) and embryo cultures (Taylor et al., 1991; Weselake et al., 1991; Taylor et al., 1992; Little et al., 1994) of *B. napus* L. In general, studies with developing seeds indicate that DGAT activity increased rapidly during the active phase of oil accumulation and then decreases markedly as seed lipid content reaches a plateau (Tzen et al., 1993; Weselake et al., 1993).

A number of studies with both mammalian (Mayorek et al., 1989; Tijburg et al., 1989) and plant (Ichihara et al., 1988; Perry and Harwood, 1993 a and 1993 b; Settlage et al. 1995) systems have suggested that DGAT may catalyze a rate-limiting reaction in TAG bioassembly. However, this hypothesis has not been rigorously tested, and has not been reduced to practice by transgenic expression of any DGAT gene in plant or animal systems, until now. Developing seeds of *B. napus* L., cv Shiralee, have been shown to produce significant levels of DAG during the active phase of oil accumulation suggesting that DGAT catalyzed reaction may regulate the flow of carbon into TAG (Perry and Harwood, 1993 a and 1993 b). In addition, an ethyl methanesulfonate-induced (EMS-induced) mutant of *A. thaliana,* designated AS11, has been shown to have a reduced DGAT activity that correlated with both an increased DAG pool and decreased accumulation of TAG (Katavic et al. 1995). Given its possible rate-limiting role in TAG bioassembly, the inventors of the present invention have identified DGAT as a potential target in the genetic modification of plant lipid biosynthesis.

Previously, the partial characterization of an EMS-induced *Arabidopis thaliana* mutant, AS11, with altered fatty acid composition was reported (Katavic et al., 1995). In comparison to wild type plant seeds, AS11 seeds have reduced levels of the very long chain fatty acid eicosenoic acid (20:1) and reduced oleic acid (18:1) and accumulate α-linolenic acid (18:3) as the major fatty acid in triacylglycerols (Figure 2). The AS11 mutant has a consistently lower ratio of TAG/DAG in developing seeds, and it accumulates an elevated amount of seed DAG (Figure 3), the substrate of the diacylglycerol acyltransferase. Through a series of biochemical analyses, it was shown that AS11 had reduced diacylglycerol acyltransferase activities throughout seed development (Figure 4). AS11 also had a reduced (by 25-30%) oil content phenotype, providing some evidence that DGAT may be controlling flux into TAG biosynthesis, as shown in Table 1 below. The AS11 did not have a wrinkled-seed phenotype as described in other low-seed-oil mutants (Focks and Benning, 1998).

**Table 1**

| Comparison of AS11 [Katavic et al., (1995) and wild-type A. thaliana seeds with respect to lipid profiles at mid-development, and the relative TAG, DAG and sterol ester contents in AS11 and WT seeds at maturity. | | | | |
|---|---|---|---|---|
| Seed Type | TAG/DAG ratio at mid-development^{a} | TAG/DAG ratio at maturity^{a} | Relative TAG content at maturity^{b} ^{c}(nmol/mg DW) | Sterol Esters at maturity (% of Totol Lipid Extract)^{d} |
| WT | 17 | 90 | 1.00^{b} ^{c}(255) | 0.8 |
| AS 11 | 5 | 20 | 0.6^{b} ^{c}(174) | 1.15 |

| | | | | |
|---|---|---|---|---|
| ^{a} Embryos staged and lipids measured as described in Katavic et al., (1995); ^{b} Relative TAG content of 200-seed samples of AS 11 and WT were meaured by MASS-1 H-NMR according to the method of Rutar (1989). The integration response for resonances attributabe to liquid-like oil were summed and the value for AS11 seed is reported relative to the response for the WT control seed sample (the latter set at a value of 1.00); ^{c}TAG content (nmoles/mg DW) measured by transmethylation of a TLC-purified TAG fraction, followed by GC analysis of fatty acid methyl esters; ^{d}A total lipid extract was prepared as dedscribed by Taylor et al., (1991; 1992), and sterol esters isolated and characterized as described in the Experimental Procedures. | | | | |

Genetic analysis indicated that the fatty acid phenotype is caused by a semidominant mutation in a nuclear gene, designated *TAG1.* The mutation was mapped to chromosome II, and was estimated to lie in the region approximately 17.5 ± 3 cM from the sti locus and 8 ± 2 cM from the cp2 locus.

Because a DGAT gene has not heretofore been cloned from any plant, until now, it has not been possible to address the possibility of genetic modifications to alter carbon flux, increase fatty acid synthesis, oil content, oil acyl composition, or seed size, by modulating plant DGAT activity.

However, there are many examples of successful modifications to plant metabolism that have been achieved by genetic engineering to transfer new genes or to alter the expression of existing genes, in plants. It is now routinely possible to introduce genes into many plant species of agronomic significance to improve crop performance (e.g. seed oil or tuber starch content/composition; meal improvement; herbicide, disease or insect resistance; heavy metal tolerance etc.) (MacKenzie and Jain, 1997; Budziszewski et al., 1996; Somerville, 1993; Kishore and Somerville, 1993).

For example, increases in the proportions of some strategic fatty acids and in the quantities of seed oil have been achieved by the introduction of various fatty acid biosynthesis and acyltransferase genes in oilseed crops. These include the following demonstrations: Expression of an anti-sense construct to the stearoyl-ACP Δ9 desaturase in Brassicaceae led to an increase in the stearic acid content (Knutzon et al., 1992). Expression of a medium chain fatty acyl-ACP thioesterase from California Bay, in Brassicaceae was demonstrated to increase the lauric acid (12:0) content (Voelker et al., 1992; 1996). Expression of a Jojoba β keto-acyl-CoA synthase in low erucic acid Brassicaceae led to an increase the level of erucic acid (22: 1); the effect following expression in high erucic acid cultivars was negligible (Lassner et al., 1996). Increased proportions of oleic acid in Brassica napus and in soybean have been achieved by silencing the microsomal FAD2 (Δ12) desaturase (Hitz et al., 1995; Kinney, 1995 ; 1997). Transformation of Arabidopsis thaliana and rapeseed (B. napus) with a yeast sn-2 acyltransferase resulted in seed oils with increased proportions of 22:1 and other very long-chain fatty acids and significant increases in seed oil content (Zou et al., 1997).

Starch deposition has also been altered by genetic engineering. By expression of a mutant E. coli gIgC16 gene encoding an ADP glucose pyrophosphorylase in potato tubers, an increase in starch accumulation was achieved (Stark et al., 1992).

The inventors therefore considered the DGAT gene to hold great promise for the desired modification of TAGs in plants.

The best modes for carrying out the invention will be apparent from the following description of the results of tests and experiments that have been carried out by the inventors.

The inventors chose to use the well-accepted model plant system *Arabidopsis thaliana* for the cloning of DGAT, as a host system for genetic engineering to alter DGAT expression, and to study the effects of altering DGAT expression on seed triacylglycerol bioassembly. This is because, over the past several years, *Arabidopsis thaliana,* a typical flowering plant, has gained increasing popularity as a model system for the study of plant biology. As a result of the ease with which this plant lends itself to work in both classical and molecular genetics, *Arabidopsis* has come to be widely used as a model organism in plant molecular genetics, development, physiology and biochemistry (Meyerowitz and Chang, 1985; Meyerowitz, 1987; Goodman et al., 1995). This model dicotyledonous plant is also closely related to *Brassica* crop species and it is increasingly apparent that information concerning the genetic control of basic biological processes in *Arabidopsis* will be transferable to other species (Lagercrantz et al., 1996).

Indeed, there are numerous examples wherein studies of the molecular biology and biochemistry of a particular metabolic pathway or developmental process and the possibility of genetically engineering a plant to bring about changes to said metabolic pathway or process, has first been tested in the model plant *Arabidopsis,* and then shown to yield similar phenotypes in other plants, particularly crop plants.

For example, the extra- plastidial membrane associated oleate (18:1) Δ12 (ω-6) desaturase gene, *FAD2,* was originally studied and eventually cloned from *Arabidopsis thaliana,* by identifying the lesion found in an *A. thaliana* mutant defective in desaturating oleate to produce linoleate (18:2) on the phosphatidylcholine backbone. This resulted in a high oleic acid phenotype in the *A*. *thaliana* seed oil (Okuley et al., 1994). Genetic engineering of both soybean *(Glycine max.*) and canola *B. napus* to silence the indigenous *FAD2* gene(s) in a seed-specific manner by anti-sense or co-suppression approaches, resulted in similar high oleic acid seed oil phenotypes (Kinney, 1995; 1997).

Transgenic expression of a yeast sn-2 acyltransferase *(SLC1-1)* gene to achieve enhanced seed oil and very long-chain fatty acid content was first performed in *Arabidopsis* and later shown to yield similar phenotypes in transgenic rapeseed *(B. napus)* experiments (Zou et al., 1997). *Arabidopsis thaliana* has repeatedly shown itself to be a useful model system for metabolic engineering of metabolic pathways (e.g. lipid biosynthesis, photosynthesis) or processes (organogenesis, reproductive development etc.) common to all higher plants.

In the area of secondary metabolism/signal transduction, an anthocyanin pathway-specific transcriptional activator from the monocot maize designated as R (the myc transcription factor involved in activation of biosynthetic genes for anthocyanin production in the aleurone cells of maize kernels), was expressed in the dicot *Arabidopsis,* causing augmented anthocyanin pigmentation in the infloresecences. Subsequent expression in another dicot, tobacco *(Nicotiana tabacum*), resulted in similar floral pigmentation changes (Lloyd et al., 1992). These experiments demonstrate that whole pathways common to all flowering plants can be co-ordinately controlled through the introduction of transcriptional regulators, and that the mechanisms are common to diverse plant species.

In the context of the current invention, all plant seeds accumulate some triacylglycerol (oil) and this ubiquitous process is affected, at least in part, by the activity of a microsomal DGAT, as explained previously. Thus, many of the effects observed following genetic engineering to modulate DGAT expression in *Arabidopsis* can be expected and predicted to result in similar phenotypes when carried out in all other plants. For example, after the present invention was made, information has become available that supports the findings of the present inventors by showing that *B. napus* has a highly homologous DGAT gene (Nikiforuk et al., 1999), and thus *B. napus* is a clear target for similar genetic modifications as those shown for *A. thaliana.*

There are a number of ways by which genes and gene constructs can be introduced into plants, and a combination of plant transformation and tissue culture techniques have been successfully integrated into effective strategies for creating transgenic crop plants. These methods, which can be used in the present 5 invention, have been extensively reviewed elsewhere (Potrykus, 1991; Vasil, 1994; Walden and Wingender, 1995; Songstad et al., 1995), and are well known to persons skilled in the art. For example, one skilled in the art will certainly be aware that, in addition to *Agrobacterium*-mediated transformation of *Arabidopsis* by vacuum infiltration (Bechtold et al., 1993) or wound inoculation (Katavic et al., 0 1994), it is equally possible to transform other plant and crop species, using *Agrobacterium* Ti-plasmid-mediated transformation (e.g. hypocotyl (DeBlock et al., 1989) or cotyledonary petiole (Moloney et al, 1989) wound infection), particle bombardment/biolistic methods (Sanford et al., 1987; Nehra et al., 1994; Becker et al., 1994) or polyethylene glycol-assisted protoplast transformation (Rhodes et al., 1988; Shimamoto et al., 1989) methods.

As will also be apparent to persons skilled in the art, and as extensively reviewed elsewhere (Meyer, 1995; Datla et al., 1997), it is possible to utilize plant promoters to direct any intended up- or down-regulation of transgene expression using constitutive promoters (e.g. those based on CaMV35S), or by using promoters which can target gene expression to particular cells, tissues (e.g. napin promoter for expression of transgenes in developing seed cotyledons), organs (e.g. roots), to a particular developmental stage, or in response to a particular external stimulus (e.g. heat shock).

Particularly preferred plants for modification according to the present invention include *Arabidopsis thaliana,* borage (*Borago* spp.), Canola, castor *(Ricinus communis),* cocoa bean *(Theobroma cacao),* corn *(Zea mays),* cotton *(Gossypium* spp), *Crambe* spp., *Cuphea* spp., flax *(Linum* spp.), *Lesquerella* and *Limnanthes* spp., Linola, nasturtium *(Tropaeolum* spp.), *Oenothera* spp., olive (*Olea* spp.), palm (*Elaeis* spp.), peanut *(Arachis* spp.), rapeseed, safflower (*Carthamus* spp.), soybean *(Glycine* and *Soja* spp.), sunflower *(Helianthus* spp.), tobacco *(Nicotiana* spp.), *Vernonia* spp., wheat *(Triticum* spp.), barley *(Hordeum* spp.), rice (*Oryza* spp.), oat *(Avena* spp.) sorghum *(Sorghum* spp.), rye *(Secale* spp.) or other members of the *Gramineae.*

The present invention is particularly useful when used to modify the yield or composition of oilseed produced from oilseed crops. Oilseed crops are plant species that are capable of generating edible or industrially useful oils in commercially significant yields, and include many of the plant species listed above. Such oilseed crops are well known to persons skilled in the art.

### RESULTS

### Isolation of the TAG1 (DGAT) cDNA from Arabidopsis thaliana

Since one of the most likely defects in AS11 mutant is at the DGAT itself (Table 1; Fig 4), the inventors attempted cloning strategies based on sequence 0 information of enzymes that share common substrates with DGAT. One of the candidate enzymes that would serve this purpose is the acyl-CoA: cholesterol acyltransferase (ACAT, EC 2.3.1.26) (Chang et al., 1997). Like DGAT, ACAT is an ER protein functioning as an O-acyltransferase by using acyl-CoA as the fatty acyl donor for the esterification of free cholesterol to generate sterol esters. Through a BLAST database search, the inventors identified an *Arabidopsis thaliana* expressed sequence tag (EST) [accession no. AA042298; SEQ ID NO:6] with a deduced amino acid sequence showing 41 % identity to that of the yeast acyl-CoA: cholesterol acyltransferase (Yang *et al.,* 1996, Yu *et al.,* 1996), within the short sequence (104 amino acids) that was available for the EST.

The corresponding cDNA (E6B2T7) clone was obtained from the Arabidopsis Biological Resource Center, Columbus, Ohio. Upon complete sequencing, the 878 bp E6B2T7 clone was found to be a partial cDNA. However, the ORF prediction from this partial cDNA confirmed the initial EST search results in that the encoded product is structurally similar to ACAT, especially in the regions at the C-terminus. The inventors were confident that the cDNA contained the 3' untranslated region through an ORF search, although the polyA tail was missing.

The inventors further used the partial cDNA sequence to search against *Arabidopsis thaliana* genomic sequence information. An *Arabidopsis'IGF'* BAC clone 'F27F23' [accession no. AC003058] was identified to include a region that matched the cDNA, and therefore it was concluded that this was the region encompassing the corresponding gene. Moreover, this BAC clone 'F27F23', is contained in the YAC clone, CIC06E08, which, according to the published map position (http://weeds.mgh.harvard.edu/goodman/c2_b.html), represents a region between centimorgan 35.9 and centimorgan 38.7 on chromosome II; this position is similar to the estimated location for *TAG1,* and the lesion identified by the mutation in AS11 (Katavic et *al.,* 1995). In view of our previous results on the characterization of the AS11 mutant, the map position of this gene strongly suggested that it may encode a DGAT.

To clone a full-length cDNA, a series of oligonucleotide primers were designed, based on the genomic sequences located at different positions 5' upstream of the region covering the partial cDNA. We used these primers in combination with a primer located at the 3' UTR of the partial cDNA (E6B2T7) to perform PCR reactions with cDNA phagemid prepared from an Arabidopsis thaliana (ecotype Columbia) silique-specific cDNA library (Giraudat et al., 1992) as a template. The longest cDNA amplified was 1904 bp, which we subsequently designated as TAG1, and deposited into the Genbank under accession AJ238008 [SEQ ID NO:1]. We believe this cDNA represents a full-length clone because its size is in agreement with that of the transcript detected in the northern blot (see Figure 6 a). The longest open reading frame is flanked by a 134-nucleotide 5' untranslated region and a 203-nucleotide 3' untranslated region. There is an in-frame stop codon (TGA at position nt-43) which is followed by an in-frame ATG at position nt-139. It is thus inferred that the ATG at position nt-139 is the initiation codon.

### The primary structure of TAG1 predicts a DGAT-related enzyme

The predicted open reading frame of the *TAG1* cDNA encodes for a polypeptide of 520 amino acids with a calculated molecular weight of 58993 Daltons. With the BLAST search program (Altschul *et al.* 1990), it was found that the recently reported mouse diacylglycerol acyltransferase [accession no. AF078752] (Cases *et al.,* 1998) is a protein which showed the highest sequence similarity to the deduced amino acid sequence of *TAG1* (Figure 5a). TAG1 was also similar to a human acyl CoA: cholesterol acyltransferase-related enzyme [accession no. AF059202]. The human acyl CoA: cholesterol acyltransferase-related enzyme, also known as ARGP1, is most likely to be a DGAT with no significant ACAT activity, although the true nature of the enzyme awaits further confirmation (Oelkers et *al.,* 1998). The similarity between TAG1 and the mammalian DGAT extends over a region of more than 400 amino acids with a sequence identity of about 41 %. A putative diacylglycerol/phorbol ester-binding motif, HKW-X-X-RH-X-Y-X-P, a signature sequence observed to be unique to DGAT while absent in the ACATs (Oelkers *et al*., 1998), is located at amino acids 414-424 ([SEQ ID NO: 7]; Figure 5 a). This diacylglycerol binding motif is also found in the subsequently published *B. napus* DGAT sequences (Nikyiforuk et al, 1999; GenBank /EMBL Accession Nos. AF155224, SEQ ID NO:8; AF164434, SEQ ID NO:9). Among other cloned acyltransferases (e.g. GPATs, LPATs, dihydroxyacetone phosphate acyltransferases) it has been reported that there is an invariant proline in a highly hydrophobic block IV that may participate in acyl-CoA binding (Lewin et al., 1999). In the TAG1 sequence, the hydrophobic block from residues 221-229 containing an invariant proline at residue 224, might constitute such a motif.

TAG1 showed some sequence similarity to other acyl CoA: cholesterol acyltransferases from a number of species (Chang et al., 1997). However, the similarity is largely confined to the C-terminus and is lower (around 30%) than is the similarity of TAG1 to the mammalian DGAT.

The TAG1 protein has multiple hydrophobic domains (Figure 5b) and an analysis by the PC Gene program predicted that the protein has 5 possible transmembrane segments (amino acids 178-195, 233-253, 363-388, 433-476, 486-507). In the mammalian DGAT, a putative tyrosine phosphorylation motif was observed (Cases *et al.,* 1998), but no apparent tyrosine phosphorylation site could be found in TAG1. However, a visual examination revealed a consensus sequence (X-L²⁰⁰-X-K²⁰²-X-X-S²⁰⁵-X-X-X-V²⁰⁹; SEQ ID NO:10) identified as a targeting motif typical of members of the SnRK1 protein kinase family, with serine residue 205 being the residue for phosphorylation. The SnRK1 (SNF1-related protein kinase-1) proteins are a class of Ser/Thr protein kinases that have been increasingly implicated in the global regulation of carbon metabolism in plants (Halford and Hardie, 1998). This concensus SnRK1 targeting motif is also found in the subsequently published *B. napus* DGAT sequences (Nikyiforuk et al, 1999; GenBank /EMBL Accession Nos. AF155224; AF164434). Interestingly, similar SnRK1 targeting motifs could also be identified in the *lys*o-phosphatidic acid acyltransferases (LPATs) from coconut (Knutzon et al., 1995) and meadowfoam (Lassner et al., 1995), respectively.

### The TAG1 gene is ubiquitously expressed in Arabidopsis

Northern blot analyses were performed to investigate the expression profile of the *TAG1* gene. Total RNA was extracted from different tissues, including roots, leaves, flowers, developing siliques, young seedlings and germinating seeds. The highest steady-state level accumulation of *TAG1* transcript was in RNA isolated from germinating seeds and young seedlings (Figure 6a). *TAG1* transcripts were also detected in root, leaf and flower tissues, albeit at lower levels. Surprisingly, the *TAG1* gene is expressed in developing siliques at a level that is comparable to that of other vegetative tissues, but lower than that of germinating seeds and young seedlings. This expression profile in general is not inconsistent with the notion that DGAT is present in all plant tissues capable of TAG biosynthesis (Kwanyuan and Wilson, 1986). It has been shown in a number of plant species, including soybean and safflower, that germinating seeds actively synthesize TAGs (Ichihara and Noda, 1981; Kwanyuan and Wilson, 1986; Wilson and Kwanyuan, 1986). The relatively high level of expression in roots is also consistent with the fact that root plastids are capable of synthesizing large amounts of triacylglycerol (Sparace et al., 1992).

Southern blot hybridization (Southern, 1975) was performed with genomic DNA digested with several restriction enzymes including BglIII, EcoRI and HindIII. The *TAG1* gene has no internal BglII and HindIII site, while one internal EcoRI site exists. Our Southern analysis suggested that *TAG1* most likely represents a single copy gene in the *Arabidopsis* genome (Figure 6b).

### An insertion mutation is found in the TAG1 gene in mutant AS11

Alignment of the genomic sequence (accession no. AC003058; SEQ ID NO:3) with that of the *TAG1* cDNA [SEQ ID NO:1] revealed that the *TAG1* gene contains 16 exons and 15 introns, spanning a region of about 3.4 kb (Figure 7a). DNA containing the *TAG1* allele from AS11 was PCR-amplified and completely sequenced. The AS11 *TAG1* allele has a 147-bp insertion located at the central region of intron 2. The insertion is a duplication of a segment that is composed of 12 bp from the 3' end of intron 1, the entire sequence of exon 2 (81 bp) and 54 bp from the 5' end of intron 2 (Figure 7a).

In order to rule out the possibility of PCR artifacts, two sets of primers were used to perform further PCR amplifications. Primers A and B (see Experimental Procedures, *Primer Strategy)* located in exons 1 and 3, respectively, amplified a DNA fragment that is about 150 bp longer from AS11 (Figure 7b, lane 2) than that from the wild type (Figure 7b, lane 1). The second pair of primers, C and B (Experimental Procedures), with one to be found in both exon 2 and the insertion segment, and the other located in exon 3, generated two amplified fragments from AS11 (Figure 7b, lane 4), while only one from the wild type (Figure 7b, lane 3). Hence these results confirmed that the insertion mutation the inventors identified through sequencing, reflected the true nature of the mutation in the *TAG1* gene in the AS11 genome.

### The AS11 TAG1 transcript has an 81-bp insertion in its open reading frame

Northern blot analyses indicated that there was no difference in the expression profiles of the *TAG1* gene, between the AS11 mutant and wild type *A. thaliana.* In order to investigate the effect of the mutation at the transcript level, reverse-transcription PCR (RT-PCR) was performed to amplify the *TAG1* transcript from RNA extracted from germinating seedlings of mutant AS11. Sequencing analysis revealed that there is an 81-bp insertion composed entirely of exon 2 in the transcript from AS11. The exon 2 in the repeat is properly spliced. The alteration of the transcript thus does not disturb the reading frame. However, this additional exon 2 sequence in the AS11 transcript would result in an altered DGAT protein with the 27 amino acid insertion ¹³¹SHAGLFNLCVVVLIAVNSRLIIENLMK¹⁵⁷ [SEQ ID NO:11]. The inventors' data shows that this insertion results in a 40-70% reduction in DGAT activity throughout seed development (Katavic et al., 1995). The 81 bp insert responsible for reduced DGAT activity in AS11 is visible in the comparison of RT-PCR products (Figure 7b: Compare lane 5 (WT) and lane 6 (AS11).) The DNA aberration observed in the AS11 mutant was unexpected, since ethyl methanesulfonate (EMS) generally causes point mutations. Although we cannot rule out the possibility that this AS11 mutant was the result of a spontaneous mutation event, EMS-induced deletions and insertions have been reported in other systems (Mogami *et al.,* 1986, Okagaki *et al.,* 1991)

### The TAG1 gene insertion in Arabidopsis mutant AS11 affects seed triacylglycerol accumulation, but not sterol ester accumulation in seeds.

Because TAG1 also showed some sequence homology to acyl CoA: cholesterol acyltransferases (ACATs) from a number of species (Chang *et al.,* 1997), the inventors compared both triacylglycerol and sterol ester accumulation in seeds of the wild-type *A. **thaliana*** and AS11 mutant. While the triacylglycerol content and TAG/DAG ratios were reduced in AS11 (i.e. increased proportion of seed oil DAGs,) in contrast, the proportions of sterol esters in WT and AS11 seeds were similar, at 0.8 and 1% of the total lipid extract, respectively (Table 1). If the *TAG1* lesion affected ACAT-like activity, one might expect a reduction in seed sterol esters, but this was not observed. These results indicated that TAG1 is not involved in sterol-ester homeogenesis, and thus not an acyl CoA: sterol acyltransferase.

### TAG 1 expression in yeast.

The *TAG1* cDNA overexpressed in yeast resulted in a 3.5 to 4-fold increase in microsomal DGAT activity compared to plasmid only *(pYES2)* control transformants (Fig. 8), confirming that the *TAG1* gene product functions as a DGAT. When ¹⁴C18:1 -CoA was added to the yeast lysates, sterol esters were also labeled *in vitro* (data not shown), but there was no significant difference in the ¹⁴C-labeled sterol esters produced by lysates from the *pYES2* GAL-induced control and the *pYES2:TAG1* Gal-induced transformant. This confirms that the TAG1 product does not encode an acyl-CoA: sterol acyltransferase (like ACAT).

### Complementation of the A. thaliana AS 11 Mutant Line by Transformation with the DGA T cDNA.

The cloned full-length DGAT cDNA was used as a template for PCR amplification with the primers DGATXbaI (CTAGTCTAGAATGGCGATTTTGGA; SEQ IN NO: 12) and DGATXhoI (GCGCTCGAGTTTCATGACATCGA; SEQ ID NO:13) to provide new restriction sites on each end of the sequence as described in Experimental Procedures. A 1.6kb fragment was excised by a Xbal/Kpnl digestion and ligated into the corresponding sites of the pSE129 vector (provided by Dr. P. Covello, PBI/NRC). pSE129A is a vector derived from the plant transformation vector pRD400 (Datla et al. 1992). The vector pSE129A contains the seed-specific napin promoter and the nos terminator cloned into the EcoRI and HindIII sites of the pRD400 plasmid (Figure 9). Hence in the DGAT-pSE129A construct, the *Arabidopsis* DGAT cDNA is under the control of the napin promoter. The construct integrity was confirmed by sequencing.

The pSE129A containing the napin:DGATcDNA was introduced into A. *tumefaciens,* used to transform *A. thaliana* mutant AS11, and progeny analyzed as described in Experimental Procedures. A number of T₂ transgenic lines were isolated which complemented the fatty acid mutant phenotype found in AS11 (reduced 20:1 and elevated polyunsaturated C₁₈S), restoring the wild-type seed fatty acid profile (Figure 10). This finding confirms the nature of the lesion in AS11 and directly ties the AS11 lipid phenotype to this mutation.

### Over-Expression of the DGA T cDNA in Wild-Type A. thaliana

The cloned full-length DGAT cDNA was used as a template for PCR amplification with the primers DGATXbal (CTAGTCTAGAATGGCGATTTTGGA ; SEQ ID NO:12) and DGATXhol (GCGCTCGAGTTTCATGACATCGA; SEQ ID NO:13) to provide new restriction sites on each end of the sequence as described in Experimental Procedures. A 1.6kb fragment was excised by a Xbal/Kpnl digestion and ligated into the corresponding sites of the pSE129 vector (provided by Dr. P. Covello, PBI/NRC). pSE129A is a vector derived from the plant transformation vector pRD400 (Datla et al. 1992). The vector pSE129A contains the seed-specific napin promoter and the nos terminator cloned into the EcoRI and HindIII sites of the pRD400 plasmid (Figure 9). Hence in the DGAT-pSE129A construct, the *Arabidopsis* DGAT cDNA is under the control of the napin promoter. The construct integrity was confirmed by sequencing.

The pSE129A containing the napin:DGATcDNA was introduced into *A. tumefaciens,* used to transform wild-type *A*. *thaliana,* and progeny analyzed as described in Experimental Procedures. A number of T₂ transgenic lines were isolated which exhibited an increased oil content (Figure 11) an increased average 100-seed weight (Figure 12) and a strong linear correlation between the two traits (Figure 13).

In terms of fatty acyl composition, wild type lines contaning over-expressed DGAT cDNA showed a decrease in the total saturates, and increases in the monounsaturates and in the 18:1/[18:2 + 18:3] index, as shown in Table 2 below. Such changes are all towards a 'healthier" oil profile, and can be applied directly to canola, other oilseeds in the *Brassicaceae* and other edible oil crops to produce similar oil composition improvements.

**Table 2**

| Fatty acid composition of seed oil from *A. thaliana* non-transformed wild-type controls (WT Con) and three T2 transgenic lines (2', 9 and 11) of wild type transformed with the DGAT cDNA under the control of a napin promoter (napin: DGAT). | | | |
|---|---|---|---|
| Line | Total Saturates^{a} Wt % | Monounsaturates^{b} Wt % | 18:1/[18:2 + 18:3] index^{c} |
| WT Control | 15.1 ± 0.1 | 36.7 ± 0,2 | 29.9 ± 0.6 |
| 2'napin:DGAT | 13.4 | 38.6 | 34.1 |
| 9 napin:DGAT | 13.1 | 39.3 | 35.6 |
| 11 napin:DGAT | 13.1 | 38.3 | 33.0 |

| | | | |
|---|---|---|---|
| ^{a} Includes 16:0, 18:0, 20:0, 24:0 ^{b} includes 18:1, 20:1, 22:1, 24:1 ^{c} ([Wt % 18:1] + [Wt % 18:2 + Wt % 18:3]) x 100 | | | |

### EXPERIMENTAL PROCEDURES

### Plant Material

*Arabidopsis thaliana* ecotype Columbia and mutant AS11 were grown under conditions described previously (Katavic et al., 1995). The A. thaliana mutant line AS11 was generated and characterized relative to wild type (WT) A. thaliana ecotype Columbia, as described by Katavic et al., (1995); (ATCC NO: PTA-1013).

### DNA manipulation

Standard methods and procedures were used for DNA preparation, plasmid propagation and isolation (Sambrook *et al.,* 1989). Sequencing was conducted on an Applied Biosystems Model 373A DNA Sequencing System using the *Taq* DyeDeoxy^{™} Terminator Cycle Sequencing Kit (Applied Biosystems, Inc.). The nucleotide and the deduced amino acid sequences were compared with sequences available in databanks using the BLAST program (Altschul *et al.,* 1990).

### Southern and Northern analysis

Total RNA was extracted from different tissues at various developmental stages, using the method of Lindstrom and Vodkin (1991). RNA samples were denatured with formaldehyde and separated on 1.2% formaldehyde-agarose gels. About 5 µg of total RNA was loaded, and the amount of RNA per lane was calibrated by the ethidium bromide-staining intensity of the rRNA bands. Genomic DNA was isolated, digested with restriction enzymes and a Southern blot analysis was performed according to Sambrook et al. (1989). The *TAG1* DNA probe was ³²P labeled by random-priming according to protocols of the manufacture (BRL).

### PCR strategy

Primers used for the amplification of the *TAG1* gene were as follows:
DGAT1 (AGACACGAATCCCATTCCCACCGA; SEQ ID NO:14), DGAT2 (AGTGGTGACAACGCAGGGATGATG; SEQ ID NO:15), DGAT3 (ATGGTCGCTCCCACATTGTGT; SEQ ID NO:16), DGAT4 (CATACAATCCCCATGACATTTATCA; SEQ ID NO:17). DGAT1 and DGAT2 amplify the 5' half of the *TAG1* gene and DGAT3 and DGAT4 amplify the 3' end of the *TAG1* gene. Genomic DNA from AS11 was used as template for PCR amplification of the mutant *TAG1* allele using the thermal profile: 94° C 3 min; 40 cycles of 94° C 30 seconds, 62° C 45 seconds, 72° C 1 min; and 72° C 15 min. To further confirm the mutation, primers A (CGACCGTCGGTTCCAGCTCATCGG: [SEQ ID NO:18]) and B (GCGGCCAATCTCGCAGCGATCTTG; [SEQ ID NO:19]), as well as primers C (TAAACAGTAGACTCATCATCG; [SEQ ID NO:20]) and B, were used in pairs, respectively, to amplify the internal fragment containing the mutation. The primers DGAT1 and DGAT4 were used for PCR amplification of the cDNA with an *A. thaliana* silique cDNA library as template. Primers A and B were also used in RT- PCR 5 amplification of the cDNA fragment encompassing the insertion segment.

### Construction of TAG1 Multicopy Vector and Transformation and Characterization of DGA T Expression in Yeast

The TAG1 cDNA was cloned into pBluescript SK as described (Hadjeb and Berkowitz, 1996). The cDNA was cut out from the vector with KpnI/XbaI, and subsquently cloned into the respective sites of the yeast expression vector pYes2 (Invitrogen). The construct was confirmed by sequencing. Constructs with TAG1 transcription under the control of the GAL1 promoter released a fragment of approximately 1.9 kb. Because the TAG1 fragment has its own initiating ATG codon, the product expressed is not a fusion protein. As a host for yeast expression, an SLC deletion strain (YMN5 [sic1Δ2::LEU2 ura3 ]) (kindly provided by M.M. Nagiec and R.C. Dickson, University of Kentucky, Lexington, KY; Nagiec et al., 1993) was used; we reasoned that in this mutant, the endogenous DAG pool may be lower than in WT yeast, and that this would allow us to maximize the activity from over-expressed TAG1 in the presence of exogenously supplied ¹⁴C-DAG during in vitro DGAT assays of transformant lysates. Yeast transformation was performed according to Elble (1992). YMN5 transformants containing vector only (pYES2) were used as controls. Single colonies were cultured overnight in 20 mL of SD medium (Synthetic Dextrose medium with glucose and without uracil, as described by Ausubel et al., 1995, Vol. 2, p. 13.1.3) on a rotary shaker (270 rpm) at 28°C. Cells were pelleted from the overnight culture and resuspended in 50 mL of medium for induction of expression (SD medium containing galactose and without uracil). Cells were reincubated at 28°C, with shaking at 270 rpm, and harvested after 4-6 hr. GAL-induced yeast transformants were harvested by centrifugation at 5000 rpm for 5 min and resuspended in 100 mM Hepes-NaOH, pH 7.4, containing 1 mM EDTA and 1 mM DTT. Cell lysates were prepared using acid-washed glass beads as described by Ausubel et al. (1995). Protein in yeast lysates was measured using the Bradford (1976) assay, protein levels in each lysate were normalized and aliquots (250 µg protein) were assayed for DGAT activity as described below.

### Lipid Substrates and DGAT Analyses

¹⁴C-labeled diolein [1-¹⁴C oleic] (Sp. activity 55 mCi/ mmol) was purchased from American Radiolabeled Chemicals (St. Louis, MO). The ¹⁴C-labelled sn-1,2-diolein isomer was purified by TLC on borate-impregnated plates and emulsified in Hepes buffer the presence of 0.2% Tween-20 as described by Taylor et al., (1991). 20:1-CoA, CoASH, ATP, and all other biochemicals were purchased from Sigma.

DGAT assays were conducted at pH 7.4, with shaking at 100 rpm in a water bath at 30°C for 30-60 min. Assay mixtures (0.5 mL final volume) contained lysate protein (250 µg), 90 mM Hepes-NaOH, 0.5 mM ATP, 0.5 mM CoASH, 1 mM MgCl₂, 200 µM sn-1,2 diolein (sp. activity 2 nC/nmol) in 0.02% Tween 20, and 18 µM 20:1-CoA as the acyl donor. The ¹⁴C-labeled TAGs were isolated by TLC and quantified as described by Taylor et al (1991 ).

### Further Lipid and Sterol Ester Analyses in AS11 and WT:

Total lipid extracts (TLEs), and lipid class analyses in WT and the AS11 mutant were performed as described by Taylor et *al.,* (1991; 1992) and by Katavic *et al.,* (1995). Relative seed oil content was also measured by magic angle sample spinning ¹H-NMR, according to the method of Rutar (1989). Analyses were conducted with 200-seed samples of intact wild-type and AS11 seeds using a Bruker AM wide-bore spectrometer (Bruker Analytische Masstechnik GHBH, Silberstreifen D-76287, Rheinstetten4/Karlstuhe, Germany) operating at 360 MHz. To reduce anisotropic line broadening, the seed sample was rotated at 1 kHz in a zirconium rotor oriented 54.7° to the magnetic field. The integration response for resonances attributable to liquid-like oil were summed and the value for AS11 seed was recorded relative to the response for the WT control seed sample, the latter set at a value of 1.00.

Sterol esters were purified from the TLEs by thin layer chromatography (TLC) on Silica H plates developed in hexane: diethyl ether: formic acid (80:20:2, v/v/v). After elution from the silica H with chloroform: methanol (2:1, v/v), the sterol esters were quantified by saponification followed by methylation of the resulting fatty acids with 3N methanolic-HCI. The fatty acid methyl esters (FAMEs) were analyzed by GC as described previously (Taylor et al., 1991). The free sterols released by saponification were also analyzed by GC on a 30 m DB-5 column; GC temperature program : initial temp: 180°C, increasing at 10°C/min to 300°C and held at this temperature for 15 min. The sterol ester content was reported as a % of the TLE; i.e. FAMEs released from sterol esters calculated as proportion of the FAMEs released by transmethylation of the total lipid extract (TLE).

### Construction of Plant Transformation Vector Containing the Wild-Type DGAT gene for Over-Expression in WT A. thaliana and Complementation of the A. thaliana AS11 Mutant:

Two primers:
Gen 1 (GAGAGGATCCACGCTCACGACCCATTCTTCCCG; [SEQ ID NO:21]), and
Gen 2 (AAGAAGGATCCATCCCCAAAACGGGACCACCAA; [SEQ ID NO:22])
were synthesized according to sequences upstream and downstream of the TAG1 gene. These primers were used to PCR amplify a genomic fragment of 5.1 kb from wild-type A. thaliana. The PCR fragment was purified and digested with BamHI and inserted into the corresponding site in plasmid pRD400 (Datla et al. 1992) to generate the plant transformation vector DGATg-pRD400.

### Construction of DGA T cDNA Plant Transformation Vector for Seed-Specific Expression:

The cloned full-length DGAT cDNA was used as a template for PCR amplification with the primers DGATXbaI (CTAGTCTAGAATGGCGATTTTGGA; SEQ ID NO:12) and DGATXhoI (GCGCTCGAGTTTCATGACATCGA; SEQ ID NO: 13) to provide new restriction sites on each end of the sequence. The PCR profile was as follows: 94°C 1 min; 30 cycles of 94°C 30 seconds, 55°C 30 seconds, 72°C 1 min; and 72°C 5 min. The PCR product was then ligated into the PCR-2.1 vector (InVitrogen). A 1.6kb fragment was excised by a Xbal/Kpnl digestion and ligated into the corresponding sites of the pSE129 vector (provided by Dr. P. Covello, PBI/NRC). pSE129A is a vector derived from the plant transformation vector pRD400 (Datla et al. 1992). The vector pSE129A contains the seed-specific napin promoter and the nos terminator cloned into the EcoRI and HindIII sites of the pRD400 plasmid (See Figure 9). Hence in the DGAT-pSE129A construct, the *Arabidopsis* DGAT cDNA is under the control of the napin promoter. The construct integrity was confirmed by sequencing.

### Transformation of Agrobacterium with Plant DGAT Vector Constructs:

Electrocompetent *Agrobacterium* cells, GV3101 (pMP90) strain, were prepared as follows: An *Agrobacterium* culture was grown 24 to 48 hrs in 2YT, and when the absorbance at 600 nm reached 0.5 to 0.7, the cells were chilled on ice and pelleted by centrifugation (5,000 x g, 10 min in a GSA rotor at 4°C). The pellet was washed in 1, 0.5 and 0.02 volumes of cold 10% sterile glycerol and resuspended in 0.01 volume of cold 10% glycerol. The electrocompetent cells were then frozen in liquid N₂ and stored at -70°C. The *Agrobacterium* cells were transformed by electroporation with 20-50 ng of transforming DNA (either DGATg-pRD400 or DGAT-pSE129A) according to the manufacturer's instructions, plated on a selective medium (LB with 50 µg/mL kanamycin) and incubated overnight at 28°C. Single transformed cells were grown overnight (28°C, 225 r.p.m.) in 5 mL LB with 50 µg/mL Kanamycin and 25 µg/mL Gentamycin. DNA extraction and purification were performed with a Qiaprep Spin Miniprep kit (Qiagen). The fidelity of the construct was re-checked by DNA sequencing before plant transformation.

### Transformation of Arabidopsis thaliana:

The transformation protocol was adapted from that described by Clough and Bent (1998). Seeds of *Arabidopsis thaliana* ecotype Columbia and mutant AS11 (Katavic et al., 1995) were grown at 22°C under fluorescent illumination (120 µE.m⁻²·s⁻¹) in a 16 h light/8 hour dark regime. Typically, four to six plants were raised in a 10 cm² pot in moistened Terra-lite Redi-earth (W. R. Grace & Co. Canada Ltd. Ajax, ON, Canada). To prevent the soil mix in the pot from falling into the inoculation media, soil was mounded as a platform with seeds sown on top, and the whole pot covered by a nylon window screen and secured by a rubber band. Plants were vacuum infiltrated in an *Agrobacterium* suspension when the first flowers started opening.

To grow *Agrobacterium,* a 5 mL suspension in LB medium containing 50 µg/mL kanamycin and 25 µg/mL gentamycin was cultured overnight at 28°C. The day before infiltration, this "seed culture" was divided into four flasks containing 250 mL of LB medium supplemented with 50 µg/mL kanamycin and 25 µg/mL gentamycin. These culture were grown overnight at 28°C. The next morning after the absorbance at 600 nm was checked (approximately = 1.0), the cells were harvested by centrifugation (5,000 x g, 10 min in a GSA rotor at room temperature) and resuspended in the infiltration medium (sucrose 5%; Silwet-77 0.005% in water) to obtain an optical density at 600 nm of 0.8. The *Agrobacterium* suspension was then poured into a beaker and the potted plants inverted into the beaker so that the flowers and bolts were submerged. The beaker was then placed into a large Bell jar and a vacuum drawn using a vacuum pump, until bubbles formed on the stem surfaces and the solution started to bubble slightly, and then the vacuum was released rapidly. [Note: The necessary time and pressure will vary from one lab setup to the next, but good infiltration is visibly apparent as uniformly darkened, water-soaked tissue.] Pots were removed from the beaker, laid on their side in a plastic tray and covered with a plastic dome, to maintain humidity. The following day, the plants were uncovered, set upright and allowed to grow for approximately four weeks in a growth chamber under continuous light conditions as described by Katavic et al., (1995). When the siliques were mature and dry, seeds were harvested and selected for positive transformants.

### Selection of Putative Transformants (Transgenic plants) and Analysis of Transgenic Plants:

For each construct, seeds were harvested in bulk. Seeds were surface-sterilized by submerging them in a solution containing 20% bleach and 0.01% Triton X-100 for 20 min, followed by three rinses with sterile water. Sterilized seeds were then plated by resuspending them in sterile 0.1% phytagar at room temperature (about 1 mL phytagar for every 500-1000 seeds), and then applying a volume containing 2,000-4,000 seeds onto 150 x 15 mm kanamycin selection plate. Plates were incubated for 2 days in the cold without light, and then grown for 7-10 days in a controlled environment (22°C under fluorescent illumination (120 µE·m⁻²·s⁻¹) in a 16 h light/8 hour dark regime). The selection media contain ½MSG medium, 0.8% phytagar, 3% sucrose, 50ug/mL kanamycin and 50ug/ mL Timentin. Petri dishes and lids were sealed with a Micropore™ surgical tape tape (3M Canada, London, ON, Canada). After 7-10 days, drug-resistant plants that had green leaves and well established roots within the medium were identified as transformants and at the 3-5 leaf stage, selected transformants were transplanted into flats filled with heavily moistened soil mix. Transformants were grown to maturity and mature seeds (T₂ generation as defined in Katavic et al., (1994)) were harvested from individualplants, and further analyzed.

### DNA isolation from and analysis of Transformants

Genomic DNA was isolated from individual T₁ plants following the protocol of Dellaporta et *al.,* (1983). A PCR amplification using the paired primers described previously for the DGAT cDNA or for the DGAT gene, was performed to confirm the presence of the cDNA or the gene, respectively, in the T₁ transformants. Southern analyses (Southern, 1975) were performed to select the transformants containing a single copy of the inserted fragment. DNA samples were digested with restriction enzymes (BgI II for the DGAT cDNA and Eco RI for the DGAT gene), resolved by electrophoresis on a 1% agarose gel, and Southern blotting performed using a nylon filter (Hybond-N+, Amersham) according to Sambrook *et al.* (1989). The DGAT cDNA fragment, labelled with α―[³²P] dCTP (NEN/DuPont) using the Random Primer DNA labelling kit (Gibco BRL), was used as a probe. Hybridization was performed at 60°C according to Church and Gilbert (1984). The filter was then exposed to Kodak X-OMAT-AR film.

### DEPOSIT INFORMATION

The following biological material has been deposited at the American Type Culture Collection (ATCC) of 10801 University Boulevard, Manassas, Virginia, 20110-2209, U.S.A. All of these deposits were made on behalf of the Applicant/Assignee (National Research Council of Canada) under the terms of the Budapest Treaty on the dates indicated, and have been given the accession numbers shown below.

| Deposited Material | Date of Deposit | Accession No. |
|---|---|---|
| Arabidopsis DGAT gene | November 29, 1999 | PTA-988 |
| Arabidopsis DGAT cDNA | November 29, 1999 | PTA-989 |
| Arabidopsis ASII seeds | December 3, 1999 | PTA-1013 |

The deposit receipts are shown later in this description.

### SEQUENCE LISTING FREE TEXT

The Sequence Listing provided below contains free text entries in respect of SEQ ID NOs: 12 to 22. The free text used in the Sequence Listing is repeated as follows:
- SEQ ID NO:12: Primer of DGATXbaI
- SEQ ID NO:13: Primer of DGATXhoI
- SEQ ID NO:14: Primer DGAT1
- SEQ ID NO:15: Primer DGAT2
- SEQ ID NO:16: Primer DGAT3
- SEQ ID NO: 17: Primer DGAT 4
- SEQ ID NO:18: Primer A
- SEQ ID NO:19: Primer B
- SEQ ID NO:20: Primer C
- SEQ ID NO:21: Primer Gen 1
- SEQ ID NO:22: Primer Gen 2.

A summary of all of the listed sequences is provided below for ease of review:
SEQ ID NO:1 - (pDGAT; vector containing isolated and purified deoxyribonucleic acid cDNA; ATCC No PTA-989), Genbank/EMBL Accession No. AJ238008.
SEQ ID NO:2 - The deduced amino acid sequence of the *Arabidopsis* DGAT (AtTAG1) protein.
SEQ ID NO:3 - (pgenomic DGAT; vector containing isolated and purified genomic deoxyribonucleic acid (genomic DNA) ATCC No PTA-988).
SEQ ID NO:4 - MDGAT, mouse DGAT [GenBank/EMBL Accession No. AF078752 (Cases *et al.,* 1998)].
SEQ ID NO:5 - HARGP1, human ARGP1 protein [GenBank/EMBL Accession No. AF059202; Oelkers et al., 1998].
SEQ NO:6 - *Arabidopsis thaliana* expressed sequence tag (EST) [accession no. AA042298).
SEQ ID NO:7 - A diacylglycerol/phorbol ester-binding motif found in SEQ ID NO:2, SEQ ID NO:8 and SEQ ID NO:9 (⁴¹⁴HKWMVRHIYFP⁴²⁴).
SEQ ID NO:8 - *B. napus* DGAT amino acid sequence GenBank EMBL Accession No AF155224.
SEQ ID NO:9 - *B. napus* DGAT amino acid sequence GenBank/EMBL Accession No. AF164434.
SEQ ID NO: 10 - Targeting motif typical of members of the SnRK1 protein kinase family found in SEQ ID NO:2, SEQ ID NO:8 and SEQ ID NO:9 X-L²⁰⁰-X-K²⁰²⁻X-X-S²⁰⁵-X-X-X-V²⁰⁹
SEQ ID NO:11 - A 27 amino acid insertion repeat in SEQ ID NO:2 found in the *Arabidopsis thaliana* AS11 mutant.

### ¹³¹SHAGLFNLCVVVLIAVNSRLIIENLMK¹⁵⁷

SEQ IN NO: 12 - CTAGTCTAGAATGGCGATTTTGGA (nucleotide sequence of Primer DGATXbaI).
SEQ ID NO:13 - GCGCTCGAGTTTCATGACATCGA (nucleotide sequence of Primer DGATXhoI).
SEQ ID NO:14 - AGACACGAATCCCATTCCCACCGA (nucleotide sequence of Primer DGAT1).
SEQ ID NO:15 - AGTGGTGACAACGCAGGGATGATG (nucleotide sequence of Primer DGAT2).
SEQ ID NO:16 - ATGGTCGCTCCCACATTGTGT (nucleotide sequence of Primer DGAT3).
SEQ ID NO:17 - CATACAATCCCCATGACATTTATCA (nucleotide sequence of Primer DGAT4).
SEQ ID NO:18 - CGACCGTCGGTTCCAGCTCATCGG (nucleotide sequence of Primer A).
SEQ ID NO:19 - GCGGCCAATCTCGCAGCGATCTTG (nucleotide sequence of Primer B).
SEQ ID NO:20 - TAAACAGTAGACTCATCATCG (nucleotide sequence of Primer C).
SEQ ID NO:21 - GAGAGGATCCACGCTCACGACCCATTCTTCCCG (nucleotide sequence of primer Gen 1).
SEQ ID NO:22 - AAGAAGGATCCATCCCCAAAACGGGACCACCAA (nucleotide sequence of primer Gen 2).
SEQ ID NO:23 - AS11 mutant DGAT cDNA nucleotide sequence.
SEQ ID NO:24 - AS11 mutant DGAT genomic DNA nucleotide sequence.
SEQ ID NO:25 - the deduced amino acid sequence of SEQ ID NO:23.

### REFERENCES RELEVANT TO THE CURRENT INVENTION

Altschul, S.F., Gish, W., Miller, W., Myers, E.W. and Lipman, D.J (1990) Basic local alignment search tool. *J. Mol. Biol.* 215, 403-410.
Ausubel, F.M., Brent, R., Kingston, R.E., Moore, D.D., Seidman, J.G., Smith, J.A., and Stuhl, K., eds (1995). Current Protocols in Molecular Biology, Vols 1, 2, and 3. Wiley, New York.
Bechtold, N., Ellis, J., and Pelletier, G. (1993) *In planta Agrobacterium*-mediated gene transfer by infiltration of adult *Arabidopsis thaliana* plants. *C R Acad Sci Paris*, *Sciences de la vie*/*Life sciences* 316: 1194-1199.
Becker, D., Brettschneider, R. and Lörz, H. (1994) Fertile transgenic wheat from microprojectile bombardment of scutellar tissue. *Plant J*. 5: 299-307.
Bernerth R, Frentzen M (1990) Utilization of erucoyl-CoA by acyltransferases from developing seeds of *Brassica napus* (L.) involved in triacylglycerol biosynthesis. *Plant Sci* 67: 21-28.
Bradford M.M. (1976). A rapid and sensitive method for the quantitation of microgram quantities of protein utilizing the principle of protein-dye binding. *Anal. Biochem.* 72*,* 248-254.
Budziszewski, G.J., Croft, K.P.C. and Hildebrand, D.F. (1996) Uses of biotechnology in modifying plant lipids. *Lipids* 31: 557-569.
Cao, Y-Z and Huang, AHC (1986) Diacylglycerol acyltransferase in maturing oil seeds of maize and other species. *Plant Physiol.* 82: 813-820.
Cao Y-Z, Huang AHC (1987) Acyle coenzyme A preference of diacylglycerol acyltransferase from maturing seeds of *Cuphea,* maize, rapeseed and canola. *Plant Physiol.* 84: 762-765
Cases, S., Smith, J.S., Zheng, Y-W., Myers, H.M., Lear, S.R., Sande, E., Novak, S., Collins, C., Welch, C.B., Lusis, A.J., Erickson, S.K. and Farese, R.V., JR. (1998) Identification of a gene encoding a acyl CoA: diacylglycerol acyltransferase, a key enzyme in triacylglycerol synthesis. *Proc. Nat'l. Acad. Sci. USA,* 95, 13018-13023.
Chang, T.Y., Chang, C.C.Y. and Cheng, D. (1997) Acyl-Coenzyme A: Cholesterol Acyltransferase. *Annu. Rev. Biochem.* 66, 613-38.
Church, G.M. and Gilbert, W. (1984) Genomic sequencing. Proc. Natl. Acad. Sci. USA. 81, 1991-95.
Clough, S.J. and Bent, A.F. (1998) Floral dip: a simplified method for *Agrobacterium-*mediated transformation of *Arabidopsis thaliana.* The Plant Journal 16, 735-43
Datla, R.S.S., Hammerlindl, J.K., Panchuk, B., Pelcher, L.E. and Keller, W.A. (1992) Modified binary plant transformation vectors with the wild-type gene encoding NPTII. *Gene* 211: 383-384.
Datla, R.S.S., Bekkaoui, F., Hammerlindi, J., Pilate, G., Dunstan, D.I. and Crosby, W.L. (1993) Improved high-level constitutive foreign gene expression in plants using an AMV RNA4 untranslated leader sequence. *Plant Sci.* 94: 139-149.
Datla, R., Anderson, J.W. and Selvaraj, G. (1997) Plant promoters for transgene expression. *Biotechnology Annual Review* 3: 269-296.
DeBlock, M., DeBrouwer, D. and Tenning P. (1989) Transformation of *Brassica napus* and *Brassica oleracea* using *Agrobacterium tumefaciens* and the expression of the *bar* and neo genes in the transgenic plants. *Plant Physiol.* 91: 694-701.
De Lange, P., Van Blokland, R., Kooter, J.M. and Mol, J.M.N. (1995) Suppression of flavenoid flower pigmentation genes in *Petunia hybrida* by the introduction of antisense and sense genes. *In:* Gene Silencing in Higher Plants and Related Phenomena in Other Eukaryotes. P.Meyer (Ed.), Springer-Verlag, Berlin, pp. 55-75.
Dellaporta, S.L., Wood, J. and Hicks, J.B. (1983) A plant DNA minipreparation:Version II. Plant Mol. Biol. Rep. 1, 19-21
Elble, R. (1992). A simple and efficient procedure for transformation of yeasts. *Biotechniques* 13,18-20.
Focks, N. and Benning, C. (1998) *wrinkled1:* A novel, low-seed-oil mutant of Arabidopsis with a deficiency in the seed-specific regulation of carbohydrate metabolism. *Plant Physiol.* 118, 91-101.
Frentzen M (1993) Acyltransferases and triacylglycerols. *In:* Moore, Jr. TS, editor, *Lipid Metabolism in Plants,* pp. 195-230. CRC Press, Ann Arbor,.
Giraudat, J., Hauge, B.M., Valon, C., Smalle, J., Parcy, F., Goodman, H.M. (1992) Isolation of the *Arabidopsis* AB13 gene by positional cloning. *Plant Cell* 4, 1251-1261.
Goodman, H.M., Ecker, J.R. and Dean, C. (1995) The genome of *Arabidopsis thaliana. Proc. Nat'l. Acad. Sci. USA* 92: 10831-10835.
Hadjeb N and Berkowitz GA (1996) Preparation of T-over-hang vectors with high PCR product cloning efficiency. *Biotechniques* 20: 21-22.
Halford, N.G. and Hardie, D.G. (1998) SNF1-related protein kinases: global regulators of carbon metabolism in plants? *Plant Mol. Biol.* 37, 735-748.
Hitz, W.D., Mauvis, C.J., Ripp, K.G., Reiter, R.J., DeBonte, L. and Chen, Z. (1995) The use of cloned rapeseed genes for cytoplastic fatty acid desaturases and the plastid acyl-ACP thioesterases to alter relative levels of polyunsaturated and saturated fatty acids in rapeseed oil. Proc. 9th Internat'nal Cambridge Rapeseed Congress UK, pp. 470-472.
Ichihara, K. and Noda, M. (1981) Lipid synthesis in germinating safflower seeds and protoplasts. *Phytochemistry* 20, 1245-1249.
Ichihara, K., Takahashi, T. and Fujii, S. (1988) Diacylglycerol acyltransferase in maturing safflower seeds: its influences on the fatty acid composition of the triacylglycerol and on the rate of triacylglycerol synthesis. *Biochim. Biophys. Acta* 958, 125-129.
Jorgensen, R.A. and Napoli, C.A. (1994) Genetic engineering of novel plant phenotypes. U.S. Patent No. 5283184.
Josefsson, L-G, Lenman M, Ericson ML and Rask L (1987) Structure of a gene encoding the 1.7S storage protein, napin, from *Brassica napus. J biol Chem* 262: 12196-12201.
Katavic, V., Haughn, G.W., Reed, D., Martin, M. and Kunst, L. (1994) In planta transformation of Arabidopsis thaliana. Mol. Gen. Genet. 245: 363-370.
Katavic, V., Reed, D.W., Taylor, D.C., Giblin, E.M., Barton, D.L., Zou, J-T., MacKenzie, S.L., Covello, P.S. and Kunst, L. (1995) Alteration of fatty acid composition by an EMS-induced mutation in Arabidopsis thaliana affecting diacylglycerol acyltransferase activity. Plant Physiol. 108, 399-409.
Kinney, A.J. (1995) Improving soybean seed quality. In: Induced Mutations and Molecular Techniques for Crop Improvement. International Atomic Energy Agency, Vienna, Austria., pp. 101-113.
Kinney, A.J. (1997) Genetic engineering of oilseeds for desired traits. In: Genetic Engineering, Vol. 19 (J.K. Setlow, ed.), Plenum Press, NY., pp. 149-166.
Kishore G.M. and Somerville, C.R. (1993) Genetic engineering of commercially useful biosynthetic pathways in transgenic plants. Current Opinion in Biotechnology. 4: 152-158.
Knutzon, D.S., Thompson, G.A., Radke, S.E., Johnson, W.B., Knauf, V.C., and Kridl, J.C. (1992) Modification of Brassica seed oil by anti-sense expression of a stearoylacyl carrier protein desaturase gene. Proc. Nat'l Acad. Sci. USA, 89: 2624-2628.
Knutzon, D.S., Lardizabal, K.D., Nelson,J.S., Bleibaum, J.L., Davis, H.M and Metz, J. (1995) Cloning of a coconut endosperm cDNA encoding a 1-acyl-sn-glycerol-3-phosphate acyltransferase that accepts medium chain length substrates. Plant Physiol. 109, 999-1006.
Kwanyuen, P. and Wilson, R.F. (1986) Isolation and purification of diacylglycerol acyltransferase from germinating soybean cotyledons. Biochim. Biophys. Acta 877, 238-245.
Lacey DJ, Hills MJ (1996) Heterogeneity of the endoplasmic reticulum with respect to lipid synthesis in developing seeds of Brassica napus L. Planta 199: 545-551.
Lagercrantz, U., Putterill, J., Coupland, G. and Lydiate, D. (1996) Comparative mapping in Arabidopsis and Brassica, fine scale genome collinearity and congruence of genes controlling flowering. Plant J. 9: 13-20.
Lassner, M.W., Levering, C.K., Davis, H.M. and Knutzon, D.S. (1995) Lysophosphatidic acid acyltransferase from meadowfoam mediates insertion of erucic acid at the sn-2 position of triacylglycerol in transgenic rapeseed oil. Plant Physiol. 109, 1389-1394.
Lassner, M.W., Lardizabal, K, and Metz, J.G. (1996) A jojoba β-ketoacyl-CoA synthase cDNA complements the canola fatty acid elongation mutation in transgenic plants. The Plant Cell, 8: 281-292.
Lewin TM, Wang P and Coleman RA (1999) Analysis of amino acid motifs diagnostic for the sn-glycerol-3-phosphate acyltransferase reaction. Biochemistry 38: 5764-5771.
Lindstrom J.T. and Vodkin L.O. (1991) A soybean cell wall protein is affected by seed colour genotype. Plant Cell 3:561-571
Little D, Weselake RJ, Pomeroy MK, Furukawa-Stoffer T and Bagu J (1994) Solubilization and characterization of diacylglycerol acyltransferase from microspore-derived cultures of oilseed rape. Biochem J 304: 951-958.
Lloyd, A.M., Walbot, V. and Davis, R.W. (1992) Arabidopsis and Nicotiana anthocyanin production activated by maize regulators R and C1. Science 258: 1773-1775.
MacKenzie, S.L. and Jain, R.K. (1997) Improvement of oils crops via biotechnology. Recent Res. Dev. In Oil Chem. 1: 149-158.
Mayorek, N, Grinstein I, and Bar-Tana J (1989) Triacylglycerol synthesis in cultured rat hepatocytes. The rate-limiting role of diacylglycerol acyltransferase. Eur J Biochem 182: 395-400.
Meyer, P. (1995) Understanding and controlling transgene expression. Trends in Biotechnology, 13: 332-337.
Meyerowitz, E.M. (1987) Arabidopsis thaliana. Ann. Rev. Genet. 21: 93-111.
Meyerowitz, E.M. and Chang, C. (1985) Molecular biology of plant growth and development: Arabidopsis thaliana as an experimental system. In: Developmental Biology, Vol. 5, Plenum Press, NY., pp. 353-366.
Mogami, K., O'Donnell, P.T., Bernstein, S.I., Wright, T.R.F and Emerson, C.P., JR. (1986) Mutations of the Drosophila myosin heavy-chain gene: effects on transcription, myosin accumulation, and muscle function. Proc. Nat'l. Acad. Sci. USA. 83, 1393-1397.
Mol, J.M.N., Van der Krol, A.R., Van Tunen, A.J., Van Blokland, R., De Lange, P. and Stuitje, A.R. (1990) Regulation of plant gene expression by antisense RNA. FEBS Lett. 268:427-430.
Moloney, M.M., Walker, J.M. and Sharma, K.K. (1989) High efficiency transformation of Brassica napus using Agrobacterium vectors. Plant Cell Rep. 8: 238-242.
Nagiec, M.M., Wells, G.B., Lester, R.L., and Dickson, R.C. (1993). A suppressor gene that enables Saccharomyces cerevisiae to grow without making sphingolipids encodes a protein that resembles an Escherichia coll fatty acyltransferase. J. Biol. Chem. 268, 22156-22163.
Nehra, N.S., Chibbar, R.N., Leung, N., Caswell, K., Mallard, C., Steinhauer, L. Baga, M. and Kartha K.K. (1994) Self-fertile transgenic wheat plants regenerated from isolated scutellar tissues following microprojectile bombardment with two distinct gene constructs. Plant J 5: 286-297.
Nykiforuk C, Laroche A and Weselake RJ (1999) Isolation and sequence analysis of a novel cDNA encoding a putative diacylglycerol acyltransferase from a microspore-derived cell suspension culture of Brassica napus L cv Jet Neuf (Accession No. AF155224).), Plant Physiology 120: 1207.
Oelkers, P., Behar, A., Cromley. D., Billheimer, J.T. and Sturley, S.T. (1998) Characterization of two human genes encoding acyl Coenzyme A: cholesterol acyltransferase-related enzymes. J. Biol. Chem. 273, 26765-26771.
Okagaki, R.J., Neuffer, M.G. and Wessier, S.R. (1991) A deletion common to two independently derived Waxy mutations In maize. Genetics 128, 425-431.
Okuley. J., Lightner, J., Feldmann, K., Yadav, N., Lark, E. and Browse, J. (1994) Arabidopsis fad2 gene encodes the enzyme that is essential for polyunsaturated lipid synthesis. The Plant Cell 6: 147-158.
Perry, H.Y. and Harwood, J.L. (1993a) Changes in the lipid content of developing seeds of Brassica napus. Phytochemistry 32: 1411-1415.
Perry, H.Y. and Harwood, J.L. (1993b) Use of [2-3H] glycerol precursor in radiolabelling studies of acyl lipids in developing seeds of Brassica napus. Phytochemistry 34: 69-73.
Poirier, Y., Dennis DE, Klomparens K and Somerville C (1992) Polyhydroxybutyrate, a biodegradable thermoplastic produced in transgenic plants. Science 256: 520-523.
Poirier, Y., Nawrath C and Somerville C (1995) Production of polyhydroxyalkanoates, a family of biodegradeable plastics and elastomers in bacteria and plants butyrate, a biodegradable thermoplastic produced in transgenic plants. Bio-Technology, 13: 142-150.
Poirier, Y., Ventre, G and Caldelari, D(1999) Increased flow of fatty acids toward β oxidation in developing seeds of Arabidopsis deficient in diacylglycerol acyltransferase activity or synthesizing medium-chain-length fatty acids. Plant Physiology 121: 1359-1366.
Potrykus, I. (1991) Gene transfer to plants: Assessment of published approaches and results. Annu. Rev. Plant Physiol. Plant Mol. Biol. 42: 205-225.
Radke SE, Andrews BM, Moloney MM, Crouch ML, Kridl JC, and Knauf VC (1988) Transformation of Brassica napus L. using Agrobacterium tumefaciens: developmentally regulated expression of a reintroduced napin gene. Theor. Appl. Genet. 75: 685-694.
Rhodes, C.A., Pierce, D.A., Mettler, I.J., Mascarenhas, D. and Detmer, J.J. (1988) Genetically transformed maize plants from protoplasts. Science 240: 204-207.
Rutar, V. (1989) Magic angle sample spinning NMR spectroscopy of liquids as a nondestructive method for studies of plant seeds. J. Agric. Food. Chem. 37, 67-70.
Sambrook J., Fritsch E.F. and Maniatis T. (1989) In Molecular Cloning, A Laboratory Manual, 2nd edition. Cold Spring Harbor Laboratory Press.
Sanford, J.C., Klein, T.M., Wolf, E.D. and Allen, N. (1987) Delivery of substances into cells and tissues using a particle bombardment process. J. Part. Sci. Technol. 5: 27-37.
Settlage, SH, Wilson RF and Kwanyuen, P. (1995) Localization of diacylglycerol acyltransferase to oil body associated endoplasmic reticulum. Plant Physiol. Biochem. 33: 399-407.
Shimamoto, K., Terada, R., Izawa, T. and Fujimoto, H. (1989) Fertile transgenic rice plants regenerated from transformed protoplasts. Nature 338: 274-276.
Somerville, C.R. (1993) Future prospects for genetic modification of the composition of edible oils from higher plants. Am. J. Clin. Nutr. 58 (2 Suppl.): 270S-275S.
Songstad, D.D., Somers, D.A. and Griesbach, R.J. (1995) Advances in alternative DNA delivery techniques. Plant Cell, Tissue and Organ Culture 40: 1-15.
Southern E.M. (1975) Detection of specific sequences among DNA fragments separated by gele electrophoresis. J. Mol. Biol. 98: 503-517.
Sparace S.A., Kleppinger-Sparace K.F., Stahl R.J., Xue L. and Qi, Q. (1992) Lipid biosynthesis in pea root plastids and some effects of glycolytic intermediates. In SL MacKenzie and DC Taylor, eds. Seed Oils for the Future, AOCS Press, Champaign, IL. pp 52-60.
Stark, D.M., Timmerman, K.P., Barry, G.F., Preiss, J. and Kishore, G.M. (1992) Regulation of the amount of starch in plant tissues by ADP glucose pyrophosphorylase. Science 258: 287-292.
Stobart AK, Stymne S, Höglund S. (1986) Safflower microsomes catalyse oil accumulation in vitro: A model system. Planta 169: 33-37.
Stymne, S. and Stobart, A.K. (1987) Triacylglycerol Biosynthesis. In Stumpf, P.K. ed, The Biochemistry of Plants, Academic Press, New York. 9, 175-214.
Taylor. CB. (1998) Comprehending cosuppression. The Plant Cell 9: 1245-1249.
Taylor, D.C., Weber, N., Barton, D.L., Underhill, E.W., Hogge, L.R., Weselake, R.J. and Pomeroy, M.K. (1991) Triacylglycerol bioassembly in microspore-derived embryos of Brassica napus L. cv. Reston. Plant Physiol. 97, 65-79.
Taylor, D.C., Barton, D.L., Rioux, K.P., MacKenzie, S.L., Reed, D.W., Underhill, E.W., Pomeroy, M.K. and Weber, N. (1992) Biosynthesis of acyl lipids containing very-long chain fatty acids in microspore-derived embryos of Brassica napus L. cv. Reston. Plant Physiol. 99, 1609-1618.
Tijburg, LB, Geelen, MJ and van Golde LM (1989) Rgulation of the biosynthesis of triacylglycerol, phosphatidylcholine and phosphatidylethanloamine in the liver. Biochim Biophys Acta 1004: 1-19.
Tzen TC, Cao Y, Laurent P, Ratnayake C and Huang HC (1993) Lipids, proteins and structures of seed oil bodies from diverse species. Plant Physiol. 101: 267-276.
Vasil, I.K. (1994) Molecular improvement of cereals. Plant Mol. Biol. 25: 925-937.
Vaucheret, H., Béclin C, Elmayan T, Feuerbach, F., Godon C, Morel J-B, Mourrain, P., Palauqui, J-C and Vernhettes S (1998) Transgene-induced gene silencing in plants. The Plant Journal 16: 651-659.
Voelker, T.A., Worrell, A.C.. Anderson, L., Bleibaum, J., Fan, C., Hawkins, D.J., Radke, S.E., and Davies, H.M. (1992) Fatty acid biosynthesis redirected to medium chains in transgenic oilseed plants. Science 257: 72-74.
Voelker, T.A., Hayes, T.R., Cramner, A.M., Turner, J.C., and Davies, H.M. (1996) Genetic engineering of a quantitative trait: metabolic and genetic parameters influencing the accumulation of laurate in rapeseed. The Plant Journal 9: 229-241.
Vogel, G and Browse, J (1996) Cholinephosphotransferase and diacylglycerol acyltransferase: Substrate specificities at a key branch point in seed lipid metabolism. Plant Physiol. 110: 923-931.
Walden, R. and Wingender, R. (1995) Gene-transfer and plant regeneration techniques. Trends in Biotechnology 13: 324-331.
Weselake, R.J., Taylor, DC, Pomeroy, M.K., Lawson SL, and Underhill EW (1991) properties of diacylglycerol acyltransferase from microspore-derived embryos of Brassica napus L. Phtochemistry : 30: 3533-3538.
Weselake, R.J., Pomeroy, M.K., Furukawa, T.L., Golden, J.L., Little, D.B. and Laroche, A. (1993) Developmental profile of diacylglycerol acyltransferase in maturing seeds of oilseed rape and safflower and micro-spore-derived cultures of oilseed rape. Plant Physiol. 102, 565-571.
Wilson, R.F. and Kwanyuan P. (1988) Triacylglycerol synthesis and metabolism in germinating soybean cotyledons. Biochim. Biophys. Acta 877, 231-237.
Yang, H., Bard, M., Bruner, D.A., Gleeson, A., Deckelbaum, R.J.. Aljinovic, G., Pohl, T.M., Rothstein, R. and Sturley, S.L. (1997) Sterol esterification in yeast: a two-gene process. Science 272, 1353-1356.
Yu, C., Kennedy, N.J., Chang, C.C.Y. and Rothblatt, J.A. (1996) Molecular cloning and characterization of two isoforms of Saccharomyces cerevisiae Acyl-CoA: Sterol Acyltransferase. J. Biol. Chem. 271, 24157-24163.
Zou, J-T., Katavic. V., Giblin, E.M., Barton, D.L., MacKenzie, S.L., Keller, W.A, Hu, X. and Taylor, D.C. (1997) Modification of seed oil content and acyl composition in the Brassicaceae by expression of a yeast sn-2 acyltransferase gene. The Plant Cell 9: 909-923.

## Claims

1. Isolated deoxyribonucleic acid (DNA), **characterized in that** said DNA includes a sequence according to SEQ ID NO: 1, and encodes a protein having diacylglycerol acyltransferase (DGAT) activity when expressed in plants.

2. A vector for transformation of plant cells, **characterized in that** said vector contains a deoxyribonucleic acid sequence according to SEQ ID NO: 1, and encodes a protein having DGAT activity when expressed in plants.

3. A vector for transformation of plant cells, **characterized in that** said vector contains a deoxyribonucleic acid sequence according to SEQ ID NO: 23.

4. A vector according to claim 2 or claim 3, **characterized in that** said sequence is present in said vector in a sense orientation.

5. A vector according to claim 2, **characterized in that** said sequence is present in said vector in an anti-sense orientation.

6. Plasmid pDGATcDNA of deposit number ATCC PTA-989.

7. A plant having a genome, **characterized in that** the genome contains an introduced recombinant nucleotide sequence of SEQ ID NO: 1 and encodes a protein having DGAT activity when expressed in plants.

8. A plant seed having a genome, **characterized in that** said genome contains an introduced recombinant nucleotide sequence of SEQ ID NO: 1 and encodes a protein having DGAT activity when expressed in plants.

9. A genetically transformed plant, **characterized in that** said plant has a genome that has been transformed by a vector according to claim 2 or claim 3.

10. A genetically transformed plant seed, **characterised in that** said seed has a genome that has been transformed by a vector according to claim 2 or claim 3.

11. A plant seed as claimed in claim 8 or claim 10, **characterized by** exhibiting an altered seed oil content compared to an average of at least 10 seeds of genomically-unmodified plants of the same genotype grown in identical conditions at the same time.

12. A plant seed as claimed in claim 8 or claim 10, **characterized by** exhibiting an altered diacylglycerol content in its seed oil compared to an average of at least 10 seeds of genomically-unmodified plants of the same genotype grown in identical conditions at the same time.

13. A plant seed as claimed in claim 8 or 10, **characterized by** exhibiting a seed oil with an altered fatty acyl composition compared to an average of at least 10 seeds of a genomically-unmodified plant of the same genotype grown in identical conditions at the same time.

14. A plant as claimed in claim 7 or 9, **characterized by** exhibiting an enhanced biomass compared to an average of at least 10 genomically-unmodified plants of the same genotype grown in identical conditions at the same time.

15. A seed as claimed in claim 8 or 10, **characterized by** exhibiting an enhanced biomass compared to an average of at least 10 genomically-unmodified plants of the same genotype grown under identical conditions at the same time.

16. A method of producing transgenic plants by introducing a recombinant nucleotide sequence into a genome of said plant, **characterized in that** said nucleotide sequence introduced into said genome includes SEQ ID NO: 1 and encodes a protein having DGAT activity when expressed in said plant, or includes SEQ ID NO: 23.

17. A method according to claim 16, **characterized in that** said plant is a member of the Brassicaceae.

18. A method according to claim 16, **characterized in that** said plant is selected from the group consisting of Arabidopsis thaliana, borage *(Borago* spp.), Canola, castor *(Ricinus communis),* cocoa bean *(Theobroma cacao),* com *(Zea mays),* cotton *(Gossypium* spp.), *Crambe* spp., *Cuphea* spp., flax *(Linum* spp), *Lesquerella* and *Limnanthes* spp., Linola, nasturtium *(Tropaeolum* spp.), *Oenothera* spp., olive (*Olea* spp.), palm *(Elaeis* spp.), peanut *(Arachis* spp.) rapeseed, safflower *(Carthamus* spp.), soybean *(Glycine* and *Soja* spp.), sunflower *(Helianthus* spp.), tobacco *(Nicotiana* spp.), *Vernonia* spp., wheat *(Triticum* spp.), barley *(Hordeum* spp.), rice (*Oryza* spp.), oat *(Avena* spp.) sorghum *(Sorghum* spp.), rye *(Secale* spp.) and other members of the *Gramineae.*

19. A method of changing the oil content, acyl composition or diacylglycerol/triacylglycerol proportions of the seed oil of plant seeds by introducing a sense or antisense recombinant nucleic acid construct into a plant transformation vector, using the vector to transform the genome of a plant or plant seed, and then growing the plant or plant seed and extracting the oil from the plant seed, **characterized in that** said recombinant nucleic acid sequence comprises SEQ ID NO: 1 or SEQ ID NO: 23.

20. An isolated DNA sequence, **characterized in that** said sequence comprises SEQ ID NO: 23.

21. A protein **characterized by** the amino acid sequence of SEQ ID NO: 2, and having DGAT activity when expressed in plants.

22. A protein according to claim 21, **characterized in that** said protein contains the consensus sequence of SEQ ID NO: 10.

23. Isolated deoxyribonucleic acid (DNA), **characterized in that** said DNA encodes a protein according to claim 21 or claim 22.

24. A vector for transformation of plant cells, **characterized in that** said vector contains a deoxyribonucleic acid sequence according to claim 23.

25. A genetically transformed plant, **characterized in that** said plant has a genome that has been transformed by a vector according to claim 24.

26. A genetically transformed plant seed, **characterized in that** said seed has a genome that has been transformed by a vector according to claim 24.

27. A protein having DGAT activity when expressed in plants, **characterized in that** said protein includes the consensus sequence of SEQ ID NO: 10.

## Patentansprüche

1. Isolierte Desoxyribonukleinsäure (DNA), **dadurch gekennzeichnet, dass** die DNA eine Sequenz nach SEQ ID NO: 1 beinhaltet und, wenn in Pflanzen exprimiert, ein Protein mit Diacylglycerolacyltransferase (DGAT)-Aktivität kodiert.

2. Vektor zur Transformation von Pflanzenzellen, **dadurch gekennzeichnet, dass** der Vektor eine Desoxyribonukleinsäure-Sequenz nach SEQ ID NO: 1 enthält und, wenn in Pflanzen exprimiert, ein Protein mit DGAT-Aktivität kodiert.

3. Vektor zur Transformation von Pflanzenzellen, **dadurch gekennzeichnet, dass** der Vektor eine Desoxyribonukleinsäure-Sequenz nach SEQ ID NO: 23 enthält.

4. Vektor nach Anspruch 2 oder Anspruch 3, **dadurch gekennzeichnet, dass** die Sequenz in dem Vektor in einer Sense-Orientierung vorliegt.

5. Vektor nach Anspruch 2, **dadurch gekennzeichnet, dass** die Sequenz in dem Vektor in einer Antisense-Orientierung vorliegt.

6. Plasmid pDGATcDNA der Hinterlegungssnummer ATCC PTA-989.

7. Pflanze mit einem Genom, **dadurch gekennzeichnet, dass** das Genom eine eingeführte rekombinante Nukleotidsequenz der SEQ ID NO: 1 enthält und, wenn in Pflanzen exprimiert, ein Protein mit DGAT-Aktivität kodiert.

8. Pflanzensamen mit einem Genom, **dadurch gekennzeichnet, dass** das Genom eine eingeführte rekombinante Nukleotidsequenz der SEQ ID NO: 1 enthält und, wenn in Pflanzen exprimiert, ein Protein mit DGAT-Aktivität kodiert.

9. Genetisch transformierte Pflanze, **dadurch gekennzeichnet, dass** die Pflanze ein Genom aufweist, welches durch einen Vektor nach Anspruch 2 oder Anspruch 3 transformiert wurde.

10. Genetisch transformierter Pflanzensamen, **dadurch gekennzeichnet, dass** der Samen ein Genom aufweist, welches durch einen Vektor nach Anspruch 2 oder Anspruch 3 transformiert wurde.

11. Pflanzensamen nach Anspruch 8 oder Anspruch 10, **gekennzeichnet durch** das Aufweisen eines veränderten Samenöl-Gehalts, verglichen mit einem Durchschnitt von mindestens 10 Samen von genomisch unmodifizierten Pflanzen des gleichen Genotyps, die unter identischen Bedingungen zur gleichen Zeit gezogen wurden.

12. Pflanzensamen nach Anspruch 8 oder Anspruch 10, **gekennzeichnet durch** das Aufweisen eines veränderten Diacylglycerol-Gehalts in seinem Samenöl, verglichen mit einem Durchschnitt von mindestens 10 Samen von genomisch unmodifizierten Pflanzen des gleichen Genotyps, die unter identischen Bedingungen zur gleichen Zeit gezogen wurden.

13. Pflanzensamen nach Anspruch 8 oder 10, **gekennzeichnet durch** das Aufweisen eines Samenöls mit einer veränderten Fettsäureacyl-Zusammensetzung, verglichen mit einem Durchschnitt von mindestens 10 Samen von einer genomisch unmodifizierten Pflanze des gleichen Genotyps, die unter identischen Bedingungen zur gleichen Zeit gezogen wurde.

14. Pflanze nach Anspruch 7 oder 9, **gekennzeichnet durch** das Aufweisen einer gesteigerten Biomasse, verglichen mit einem Durchschnitt von mindestens 10 genomisch unmodifizierten Pflanzen des gleichen Genotyps, die unter identischen Bedingungen zur gleichen Zeit gezogen wurden.

15. Samen nach Anspruch 8 oder 10, **gekennzeichnet durch** das Aufweisen einer gesteigerten Biomasse, verglichen mit einem Durchschnitt von mindestens 10 genomisch unmodifizierten Pflanzen des gleichen Genotyps, die unter identischen Bedingungen zur gleichen Zeit gezogen wurden.

16. Verfahren zur Herstellung transgener Pflanzen durch das Einführen einer rekombinanten Nukleotidsequenz in ein Genom der Pflanze, **dadurch gekennzeichnet, dass** die in das Genom eingeführte Nukleotidsequenz SEQ ID NO: 1 beinhaltet und, wenn in der Pflanze exprimiert, ein Protein mit DGAT-Aktivität kodiert, oder SEQ ID NO: 23 beinhaltet.

17. Verfahren nach Anspruch 16, **dadurch gekennzeichnet, dass** die Pflanze ein Mitglied der Brassicaceae ist.

18. Verfahren nach Anspruch 16, **dadurch gekennzeichnet, dass** die Pflanze aus der Gruppe, bestehend aus *Arabidopsis thaliana,* Borretsch *(Borago* spp.), Canola, Rizinus *(Ricinus communis),* Kakaobohne *(Theobroma cacao),* Mais (Zea *mays),* Baumwollpflanze *(Gossypium* spp.), *Crambe* spp., *Cuphea* spp., Flachs *(Linum* spp.), *Lesquerella* und *Limnanthes* spp., Linola, Kapuzinerkresse *(Tropaeolum* spp.), *Oenothera* spp., Olive (Olea spp.), Palme *(Elaeis* spp.), Erdnuss *(Arachis* spp.), Rübsamen, Saflor *(Carthamus* spp.), Sojabohne *(Glycine* und Soja spp.), Sonnenblume *(Helianthus* spp.), Tabak *(Nicotiana* spp.), *Vernonia* spp., Weizen *(Triticum* spp.), Gerste *(Hordeum* spp.), Reis *(Oryza* spp.), Hafer *(Avena* spp.), Sorghum *(Sorghum* spp.), Roggen (*Secale* spp.) und anderen Mitgliedern der *Gramineae,* ausgewählt ist.

19. Verfahren zum Verändern des Ölgehalts, der Acyl-Zusammensetzung oder der Diacylglycerol/Triacylglycerol-Verhältnisse des Samenöls von Pflanzensamen durch das Einführen eines rekombinanten Sense- oder Antisense-Nukleinsäure-Konstrukts in einen Pflanzen-Transformationsvektor, das Verwenden des Vektors, um das Genom einer Pflanze oder eines Pflanzensamens zu transformieren, und dann das Züchten der Pflanze oder des Pflanzensamens und das Extrahieren des Öls aus dem Pflanzensamen, **dadurch gekennzeichnet, dass** die rekombinante Nukleinsäuresequenz SEQ ID NO: 1 oder SEQ ID NO: 23 umfasst.

20. Isolierte DNA-Sequenz, **dadurch gekennzeichnet, dass** die Sequenz SEQ ID NO: 23 umfasst.

21. Protein, **gekennzeichnet durch** die Aminosäuresequenz von SEQ ID NO: 2 und, wenn in Pflanzen exprimiert, mit DGAT-Aktivität.

22. Protein nach Anspruch 21, **dadurch gekennzeichnet, dass** das Protein die Consensus-Sequenz von SEQ ID NO: 10 enthält.

23. Isolierte Desoxyribonukleinsäure (DNA), **dadurch gekennzeichnet**, das die DNA ein Protein nach Anspruch 21 oder Anspruch 22 kodiert.

24. Vektor zur Transformation von Pflanzenzellen, **dadurch gekennzeichnet, dass** der Vektor eine Desoxyribonukleinsäure-Sequenz nach Anspruch 23 enthält.

25. Genetisch transformierte Pflanze, **dadurch gekennzeichnet, dass** die Pflanze ein Genom aufweist, welches durch einen Vektor nach Anspruch 24 transformiert wurde.

26. Genetisch transformierter Pflanzensamen, **dadurch gekennzeichnet, dass** der Samen ein Genom aufweist, weiches durch einen Vektor nach Anspruch 24 transformiert wurde.

27. Protein mit DGAT-Aktivität, wenn in Pflanzen exprimiert, **dadurch gekennzeichnet, dass** das Protein die Consensus-Sequenz von SEQ ID NO: 10 beinhaltet.

## Revendications

1. Acide désoxyribonucléique (ADN) isolé, **caractérisé en ce que** ledit ADN comprend une séquence correspondant à SEQ ID NO:1 et code pour une protéine ayant une activité de diacylglycérolacyltransférase (DGAT) lorsqu'elle est exprimée dans des plantes.

2. Vecteur de transformation de cellules de plantes, **caractérisé en ce que** ledit vecteur contient une séquence d'acide désoxyribonucléique correspondant à SEQ ID NO:1 et code pour une protéine ayant une activité de DGAT lorsqu'elle est exprimée dans des plantes.

3. Vecteur de transformation de cellules de plantes, **caractérisé en ce que** ledit vecteur comprend une séquence d'acide désoxyribonucléique correspondant à SEQ ID NO:23.

4. Vecteur selon la revendication 2 ou 3, **caractérisé en ce que** ladite séquence est présente dans ledit vecteur dans une orientation sens.

5. Vecteur selon la revendication 2, **caractérisé en ce que** ladite séquence est présente dans ledit vecteur dans une orientation antisens.

6. Plasmide pDGATcDNA, déposé sous le numéro de référence ATCC PTA-989.

7. Plante au génome **caractérisé en ce qu'**il comprend une séquence nucléotidique recombinante introduite correspondant à SEQ ID NO: 1 et qu'il code pour une protéine ayant une activité de DGAT lorsqu'elle est exprimée dans des plantes.

8. Graine de plante au génome **caractérisé en ce qu'**il comprend une séquence nucléotidique recombinante introduite correspondant à SEQ ID NO:1 1 et qu'il code pour une protéine ayant une activité de DGAT lorsqu'elle est exprimée dans des plantes.

9. Plante génétiquement transformée, **caractérisée en ce que** ladite plante comporte un génome ayant été transformé par un vecteur selon la revendication 2 ou 3.

10. Graine de plante génétiquement transformée, **caractérisée en ce que** ladite graine comporte un génome ayant été transformé par un vecteur selon la revendication 2 ou 3.

11. Graine de plante selon la revendication 8 ou 10, **caractérisée en ce qu'**elle présente une teneur en huile de graine modifiée par comparaison à une moyenne d'au moins 10 graines de plantes génomiquement non modifiées de même génotype et cultivées dans des conditions identiques au même moment.

12. Graine de plante selon la revendication 8 ou 10, **caractérisée en ce qu'**elle présente dans son huile de graine une teneur en diacylglycérol modifiée par comparaison à une moyenne d'au moins 10 graines de plantes génomiquement non modifiées de même génotype et cultivées dans des conditions identiques au même moment.

13. Graine de plante selon la revendication 8 ou 10, **caractérisée en ce qu'**elle présente une huile de graine ayant une composition en acides gras modifiée par comparaison à une moyenne d'au moins 10 graines d'une plante génomiquement non modifiée de même génotype et cultivée dans des conditions identiques au même moment.

14. Plante selon la revendication 7 ou 9, **caractérisée en ce qu'**elle présente une biomasse améliorée par comparaison à une moyenne d'au moins 10 plantes génomiquement non modifiées de même génotype et cultivées dans des conditions identiques au même moment.

15. Graine selon la revendication 8 ou 10, **caractérisée en ce qu'**elle présente une biomasse améliorée par comparaison à une moyenne d'au moins 10 plantes génomiquement non modifiées de même génotype et cultivées dans des conditions identiques au même moment.

16. Procédé de production de plantes transgéniques par introduction dans un génome de ladite plante d'une séquence nucléotidique recombinante, **caractérisé en ce que** ladite séquence nucléotidique introduite dans ledit génome comprend une SEQ ID NO:1 et code pour une protéine ayant une activité de DGAT lorsqu'elle est exprimée dans ladite plante, ou comprend une SEQ ID NO:23.

17. Procédé selon la revendication 16, **caractérisé en ce que** ladite plante est un membre de la famille des Brassicacées.

18. Procédé selon la revendication 16, **caractérisé en ce que** ladite plante est choisie dans le groupe constitué *d'Arabidopsis thaliana,* de bourrache *(Borago sp.),* de canola, de ricin *(Ricinus communis),* de fève de cacao *(Theobroa cacao),* de maïs *(Zea mays),* de coton *(Gossypium sp.),* de crambe *sp.,* de *Cuphea sp.,* de lin *(Linum sp.),* de *Lesquerella sp.* et de *limnanthes sp.,* de Linola, de nasturce *(Tropaeolum sp.), d'Oenothera sp.,* d'olive *(Olea sp.),* de palme *(Elaeis sp.),* d'arachide *(Arachis sp.),* de colza, de carthame *(Carthamus sp.),* de soja *(Glycine* et *Soja sp.),* de tournesol *(Helianthus sp.),* de tabac *(Nicotiana sp.),* de *Vernonia sp.,* de blé *(Triticum sp.),* d'orge *(Hordeum sp.),* de riz *(Oryza sp.),* d'avoine *(Avena sp.),* de sorgho *(Sorghum sp.),* de seigle *(Secale sp.)* et d'autres membres de la famille des graminées.

19. Procédé de modification de la teneur en huile, de la composition en acides gras ou des proportions en diacylglycérol/triacylglycérol de l'huile de graines de graines de plantes, par introduction dans un vecteur de transformation de plante d'un construit d'acide nucléique recombinant sens ou antisens, en utilisant le vecteur pour transformer le génome d'une plante ou d'une graine de plante, puis en cultivant la plante ou la graine de plante et en procédant à l'extraction de l'huile à partir de la graine de plante, **caractérisé en ce que** ladite séquence d'acide nucléique recombinant comprend la SEQ ID NO:1 ou la SEQ ID NO:23.

20. Séquence d'ADN isolé, **caractérisée en ce que** ladite séquence comprend la SEQ ID NO:23.

21. Protéine **caractérisée par** la séquence d'acides aminés de la SEQ ID NO:2 et ayant une activité de DGAT lorsqu'elle est exprimée dans des plantes.

22. Protéine selon la revendication 21, **caractérisée en ce que** ladite protéine contient la séquence consensus de la SEQ ID NO:10.

23. Acide désoxyribonucléique isolé (ADN), **caractérisé en ce que** ledit ADN code pour une protéine selon la revendication 21 ou 22.

24. Vecteur de transformation de cellules de plantes, **caractérisé en ce que** ledit vecteur comprend une séquence d'acide désoxyribonucléique selon la revendication 23.

25. Plante génétiquement transformée, **caractérisée en ce que** ladite plante a un génome qui a été transformé par un vecteur selon la revendication 24.

26. Graine de plante génétiquement transformée, **caractérisée en ce que** ladite graine a un génome qui a été transformé par un vecteur selon la revendication 24.

27. Protéine ayant une activité de DGAT lorsqu'elle est exprimée dans des plantes, **caractérisée en ce que** ladite protéine comprend la séquence consensus de la SEQ ID NO:10.
